(19) Europäisches Patentamt
European Patent Office
Office européen des brevets

(11) **EP 4 379 446 A1**

(12) **EUROPEAN PATENT APPLICATION**
published in accordance with Art. 153(4) EPC

(43) Date of publication:
**05.06.2024 Bulletin 2024/23**

(21) Application number: **22861383.2**

(22) Date of filing: **24.08.2022**

(51) International Patent Classification (IPC):
**G02B 21/32** (2006.01)        **B25J 7/00** (2006.01)
**B25J 13/08** (2006.01)        **C12M 1/00** (2006.01)
**C12M 3/00** (2006.01)

(52) Cooperative Patent Classification (CPC):
**B25J 7/00; B25J 13/08; C12M 1/00; C12M 3/00; G02B 21/32**

(86) International application number:
**PCT/JP2022/031821**

(87) International publication number:
**WO 2023/027095 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.08.2021   JP 2021139395**
**18.05.2022   JP 2022081752**

(71) Applicant: **National University Corporation Tokai National**
**Higher Education and Research System**
**Nagoya-shi, Aichi 464-8601 (JP)**

(72) Inventors:
• **AOYAMA, Tadayoshi**
  **Nagoya-shi Aichi 464-8601 (JP)**
• **FUJISHIRO, Toshiki**
  **Nagoya-shi Aichi 464-8601 (JP)**
• **SAKAMOTO, Kazuya**
  **Nagoya-shi Aichi 464-8601 (JP)**
• **FUNABORA, Yuki**
  **Nagoya-shi Aichi 464-8601 (JP)**
• **SAITO, Sumiwa**
  **Nagoya-shi Aichi 464-8601 (JP)**

(74) Representative: **Seemann & Partner**
**Patentanwälte mbB**
**Raboisen 6**
**20095 Hamburg (DE)**

(54) **MANIPULATION SYSTEM AND MANIPULATION METHOD**

(57)     A manipulation system 10 includes: a manipulator 16 for manipulating a sample 40; a manipulator drive mechanism 26 for moving the manipulator 16; an imaging apparatus 24 for imaging the sample 40 through an objective lens 20; a control apparatus 30 that generates force information indicating a magnitude of a force sensation presented to a user, based on an image captured by the imaging apparatus 24; and a force sensation presentation apparatus 36, 80 configured to receive an input operation from the user for designating a position of the manipulator and to present a force sensation according to the force information generated by the control apparatus 30 to the user.

EP 4 379 446 A1

# FIG. 10

**Description**

TECHNICAL FIELD

The present disclosure relates to manipulation systems and manipulation methods.

BACKGROUND ART

Manipulation systems for manipulating cells by using a manipulator are known. Since cells are tiny, cells are manipulated while the positions of the cells and the manipulator are observed under a microscope. The manipulator is attached to a table capable of fine movement in three axes of XYZ, and the user moves the manipulator by using a joystick, etc.

RELATED-ART LITERATURE

PATENT LITERATURE

PATENT LITERATURE 1: JP 2020-122898

SUMMARY OF INVENTION

TECHNICAL PROBLEM

In order to manipulate a cell properly, it is necessary not only to control the position of the manipulator but also to control a force applied to the cell by the manipulator properly. While manipulating a manipulator by using a joystick, the user cannot perceive a force applied to the cell directly. An expert in cell manipulation can perform manipulation skillfully while imagining a force applied to the cell based on visual information from the microscope. In the case of beginners in cell manipulation, however, it is not easy to grasp a force applied to the cell only by visual information, and it generally takes a long time (e.g., one year or more) to acquire the skill.

The present disclosure addresses the issue described above, and an illustrative purpose is to provide a technology for improving the operability experienced when a sample is manipulated by using a manipulator.

SOLUTION TO PROBLEM

A manipulation system according to an embodiment of the present disclosure includes: a manipulator for manipulating a sample; a manipulator drive mechanism for moving the manipulator; an imaging apparatus for imaging the sample through an objective lens; a control apparatus that generates force information indicating a magnitude of a force sensation presented to a user, based on an image captured by the imaging apparatus; and a force sensation presentation apparatus configured to receive an input operation from the user for designating a position of the manipulator and to present a force sensation according to the force information generated by the control apparatus to the user.

Another embodiment of the present disclosure relates to a manipulation method. The method includes: acquiring position information based on a user's input operation for designating a position of a manipulator from a force sensation presentation apparatus; controlling, based on the position information acquired, an operation of a manipulator drive mechanism for moving the manipulator; imaging a sample manipulated by using the manipulator through an objective lens; generating, based on an image captured, force information indicating a magnitude of a force sensation presented to a user; and controlling an operation of the force sensation presentation apparatus so that a force sensation according to the force information generated is presented to the user.

Still another embodiment of the present disclosure relates to a manipulation system. The manipulation system includes: a manipulator for manipulating the sample; a manipulator drive mechanism for moving the manipulator; a pump that variably controls a suction force and a discharge force at a leading end of the manipulator; an input operation apparatus including a holding member held by a user, a link mechanism that supports the holding member so that a position of the holding member is variable according to a user operation, a position sensor for detecting the position of the holding member, a rotating member that rotates with respect to the holding member according to a gripping action of the user, and an angle sensor for detecting a rotation angle of the rotating member; and a control apparatus that controls an operation of the manipulator drive mechanism based on position information indicating the position of the holding member detected by the position sensor and controls an operation of the pump based on angle information indicating the rotation angle of the rotating member detected by the angle sensor.

Still another embodiment of the present disclosure relates to a manipulation method. The method includes:

acquiring position information indicating a position of a holding member held by a user and supported by a link mechanism so that the position is variable according to a user operation; acquiring angle information indicating a rotation angle of a rotating member that rotates with respect to the holding member according to a gripping action of the user; controlling, based on the position information acquired, an operation of a manipulator drive mechanism for moving a manipulator for manipulating a sample; and controlling an operation of a pump that variably controls a suction force and a discharge force at a leading end of the manipulator, based on the angle information acquired.

Optional combinations of the aforementioned constituting elements, and mutual substitution of constituting elements and implementations of the present disclosure between methods, programs, etc. may also be practiced as additional modes of the present disclosure.

ADVANTAGEOUS EFFECTS OF INVENTION

According to the present disclosure, the operability experienced when a sample is manipulated by using a manipulator is improved.

BRIEF DESCRIPTION OF DRAWINGS

[FIG. 1] FIG. 1 is a diagram schematically showing a configuration of the manipulation system according to the first embodiment.
[FIG. 2] FIG. 2 is a diagram schematically showing the working distance identified when the influence of the refractive index is considered.
[FIG. 3] FIG. 3 is a diagram schematically showing a method for identifying the position coordinate of the sample in the z direction.
[FIG. 4] FIG. 4 is a graph showing a correlation between the average edge intensities and the working distance approximated by a Gaussian distribution.
[FIG. 5] FIG. 5 is a diagram schematically showing a measurement pattern for the manipulator for deriving coordinate conversion parameters.
[FIG. 6] FIGS. 6A-6J are diagrams showing images captured in the field of view of the objective lens and examples displayed on the three-dimensional display apparatus.
[FIG. 7] FIG. 7 is a flowchart showing a three-dimensional position presentation method according to the embodiment.
[FIG. 8] FIGS. 8A-8C are diagrams schematically showing how the sample is manipulated by the manipulator.
[FIG. 9] FIG. 9 is a flowchart showing a force sensation presentation method according to the embodiment.
[FIG. 10] FIG. 10 is a diagram schematically showing a configuration of the manipulation system according to the second embodiment.
[FIG. 11] FIGS. 11A-11B are diagrams schematically showing a configuration of the second force sensation presentation apparatus.
[FIG. 12] FIG. 12 is a diagram schematically showing a method for determining a contact between the sample and the manipulator.
[FIG. 13] FIG. 13 schematically shows how the sample is manipulated by the manipulator.
[FIG. 14] FIG. 14 schematically shows how the sample is manipulated by the manipulator.
[FIG. 15] FIG. 15 schematically shows how the sample is manipulated by the manipulator.
[FIG. 16] FIG. 16 is a flowchart showing an example of the manipulation method according to the present disclosure.
[FIG. 17] FIG. 17 is a flowchart showing an example of the manipulation method according to the present disclosure.
[FIG. 18] FIG. 18 is a diagram schematically showing a configuration of the manipulation system according to the third embodiment.
[FIG. 19] FIG. 19 is a diagram schematically showing a configuration of the third force sensation presentation apparatus.
[FIG. 20] FIG. 20 is a diagram schematically showing the amount of deformation at a plurality of positions of the sample manipulated by the manipulator.
[FIG. 21] FIG. 21 is a diagram schematically showing a method for calculating the amount of deformation of the sample by using an optical flow.
[FIG. 22] FIGS. 22A-22C are diagrams schematically showing a piercing operation on the sample by the manipulator.

DESCRIPTION OF EMBODIMENTS

(First embodiment)

First, an overview of the present disclosure will be described. The present disclosure relates to a manipulation system. The manipulation system includes an optical microscope for observing a sample such as a cell and a manipulator for manipulating the sample. The user manipulates the sample by moving the manipulator while observing the sample and the manipulator with an optical microscope. The position of the manipulator is controlled by a drive mechanism such as an actuator to enable micromanipulation in units of micrometers. In order to manipulate cell properly, it is necessary to control a force applied to the cell properly. However, the user cannot directly feel a force applied to the cell by the manipulator. In addition, the force applied to the cell by the manipulator is very small, i.e., on the order of 1 $\mu$N-1 mN (0.1 mgf-0.1 gf), and it is difficult to detect the force in real time by using a sensor, etc.

In the present disclosure, the amount of change in at least one of the position and shape of the sample manipulated by the manipulator is identified in real time, and a force applied to the sample is estimated based on the identified amount of change. Further, by amplifying the estimated force by about 100 times-10,000 times and feeding back the estimated force through a force sensation device, the user can feel the reaction force applied to the manipulator when manipulating the sample. According to the present disclosure, it is possible to present a force sensation as if the cell is directly manipulated by hand so that the operability of the manipulation system can be enhanced. In particular, it can help beginners with little experience in cell manipulation to acquire the skill.

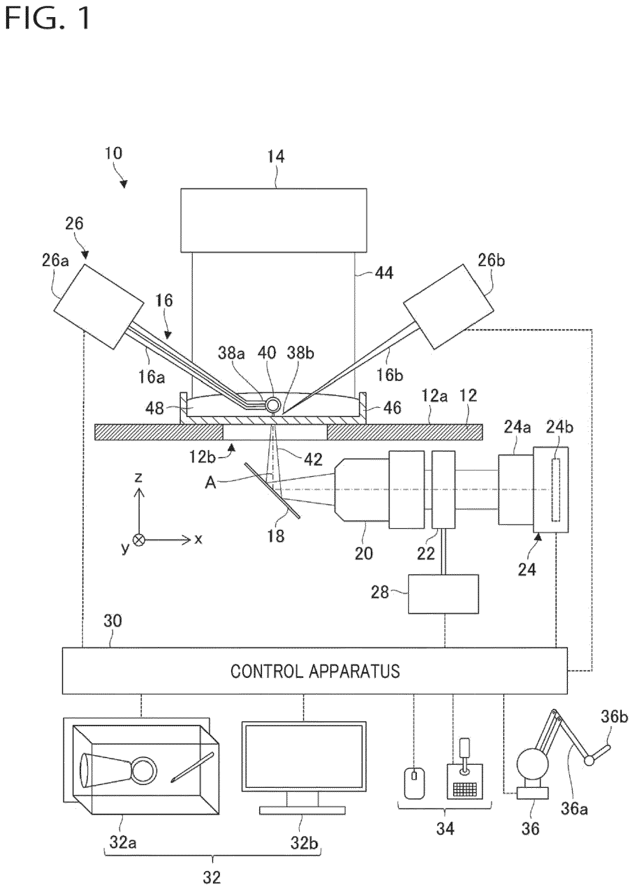

FIG. 1 is a diagram schematically showing a configuration of a manipulation system 10 according to the first embodiment. The manipulation system 10 includes an inverted microscope configuration. The manipulation system 10 includes a stage 12, an illumination apparatus 14, a manipulator 16, a redirecting mirror 18, an objective lens 20, a variable focus lens 22, an imaging apparatus 24, a manipulator drive mechanism 26, a lens drive mechanism 28, a control apparatus 30, a display apparatus 32, an input apparatus 34, and a force sensation presentation apparatus 36.

Referring to FIG. 1, a first coordinate system is set with reference to an optical axis A of the objective lens 20 on the stage 12. The direction in which the optical axis A of the objective lens 20 extends on the stage 12 is the z direction, and the directions perpendicular to the optical axis A are the x direction and the y direction. In the illustrated example, the direction in which the optical axis A of the objective lens 20 extends on the stage 12 coincides with the direction perpendicular to a support surface 12a of the stage 12. It should be noted that the direction in which the optical axis A of the objective lens 20 extends on the stage 12 may be deviated from the direction perpendicular to the support surface 12a of the stage 12.

The stage 12 has a support surface 12a for horizontally supporting the sample 40 and an opening 12b for transmitting an observation light 42 from the sample 40. The sample 40 subject to manipulation does not particularly matter, but cells such as those of humans and animals can be manipulated. The sample 40 is, for example, contained in a sample dish 46 made of a transparent material such as resin and glass, and the sample dish 46 is arranged on stage 12. The sample 40 is, for example, suspended in a liquid 48 such as water contained in the sample dish 46.

The illumination apparatus 14 is provided above the stage 12 and illuminates the sample 40 on the stage 12. The illumination apparatus 14 projects an illumination light 44 such as white light toward the sample 40. The illumination apparatus 14 is configured to provide transillumination. The illumination apparatus 14 may be capable of projecting a visible illumination light 44 of a specific wavelength selected for fluorescent observation, etc. The illumination apparatus 14 projects, for example, the illumination light 44 that provides a uniform illuminance distribution on the stage 12.

The manipulator 16 is provided on the stage 12 and is used to manipulate the sample 40. In the illustrated example, the manipulator 16 includes a holding pipette 16a and an injection pipette 16b. For example, the holding pipette 16a is used to fix a cell, and the injection pipette 16b is used for cell manipulation such as gene transfer into the cell. In the illustrated example, two manipulators are provided, but the number of manipulators may be one or three or more.

The redirecting mirror 18 is provided directly below the opening 12b of the stage 12. The redirecting mirror 18 is arranged so as to reflect the observation light 42 from the sample 40 toward the objective lens 20. In the illustrated example, the optical axis A of the objective lens 20 is folded back by the redirecting mirror 18, but the redirecting mirror 18 may not be provided and the objective lens 20 may be arranged on an optical axis extending in the z direction.

The objective lens 20 is arranged at a position where the observation light 42 from the redirecting mirror 18 is incident. The objective lens 20 is arranged at a position distanced from the redirecting mirror 18 in the +x direction. The objective lens 20 preferably has a relatively long working distance (WD). The specification such as magnification factor and working distance of the objective lens 20 is not particularly limited. For example, an ultra-long working objective lens having a working distance of 20 mm-40 mm at a magnification factor of 10 times-50 times can be used.

The variable focus lens 22 is arranged at a position where the observation light 42 transmitted through the objective lens 20 is incident. The variable focus lens 22 is arranged between the objective lens 20 and the imaging apparatus 24 and is arranged, for example, adjacent to or in close proximity to the objective lens 20. The variable focus lens 22 is configured to have refractive power that is variable within a predetermined range. The variable focus lens 22 may be a convex lens having only positive refractive power, a concave lens having only negative refractive power, or may be configured to have positive or negative refractive power switchably.

The variable focus lens 22 is composed of, for example, a liquid lens, and is configured so that the focal length is variable by deforming a flexible transparent membrane that seals the liquid lens. The shape of the transparent membrane is controlled by varying the pressure applied to the transparent membrane. For example, the focal length of the variable focus lens 22 can be electrically controlled by using an electromagnetic actuator or a piezoelectric device. The variable focus lens 22 is configured, for example, so that the effective working distance in the combination of the objective lens 20 and the variable focus lens 22 is variable in a range of about 2 mm.

The imaging apparatus 24 generates a captured image by imaging the observation light 42 transmitted through the variable focus lens 22. The imaging apparatus 24 includes an imaging lens 24a and an imaging device 24b. The imaging lens 24a forms an image of the observation light 42 on the imaging device 24b. The imaging device 24b is an image sensor such as a CMOS sensor and is capable of generating a captured image at a high frame rate. The frame rate of the imaging apparatus 24 is not particularly limited but is preferably 100 frames per second or more and, more preferably, 500 frames per second or more.

The objective lens 20, the variable focus lens 22, and the imaging apparatus 24 are arranged along the optical axis A extending in the x direction and are fixed to, for example, a lens barrel extending in the x direction. An additional redirecting mirror not shown may be provided between the variable focus lens 22 and the imaging apparatus 24 to further fold back the optical axis A.

The manipulator drive mechanism 26 moves the manipulator 16 to make the three-dimensional position of the manipulator 16 variable. In the illustrated example, the manipulator drive mechanism 26 includes a first drive mechanism 26a and a second drive mechanism 26b. The first drive mechanism 26a is configured to move the holding pipette 16a to make the three-dimensional position of the holding pipette 16a variable. The second drive mechanism 26b is configured to move the injection pipette 16b to make the three-dimensional position of the injection pipette 16b variable. The three-dimensional position of each of the holding pipette 16a and the manipulator 16 is controllable independently of each other.

The lens drive mechanism 28 drives the variable focus lens 22 and changes the refractive power of the variable focus lens 22. The lens drive mechanism 28 changes the effective working distance in the combination of the objective lens 20 and the variable focus lens 22 by changing the refractive power of the variable focus lens 22. The effective working distance is a distance from the leading end of the objective lens 20 to the focal position of the observation light 42 and is a distance from the leading end of the objective lens 20 to the focal plane where the image captured by the imaging apparatus 24 is focused.

The control apparatus 30 controls the overall operation of the manipulation system 10. The control apparatus 30 is implemented in hardware such as devices and mechanical apparatuses exemplified by a CPU and a memory of a computer, and in software such as a computer program. The control apparatus 30 is comprised of, for example, of a general-purpose personal computer.

The display apparatus 32 includes a three-dimensional display apparatus 32a and a two-dimensional display apparatus 32b. The three-dimensional display apparatus 32a displays the three-dimensional positions of the sample 40 and the manipulator 16 in three dimensions. The three-dimensional display apparatus 32a is, for example, a hologram display such as Looking Glass and is a display apparatus that enables stereoscopic vision without using 3D glasses, etc. A computer graphic (CG) image that simulates the sample 40 and the manipulator 16 is displayed on the three-dimensional display apparatus 32a. The three-dimensional display apparatus 32a may be a head-mounted virtual reality (VR) display apparatus.

The two-dimensional display apparatus 32b is a liquid crystal display, etc. and displays an image captured by the imaging apparatus 24 in real time. The two-dimensional display apparatus 32b may display the three-dimensional positions of the sample 40 and the manipulator 16 and may display a rendered image generated by perspective projection of the sample 40 and the manipulator 16, mapped to a virtual space, onto an arbitrary observation surface.

The input apparatus 34 is an apparatus for providing an input to the control apparatus 30 and to manipulate the manipulator 16. A mouse, a keyboard, etc. can be used as a means to provide an input to the control apparatus 30. A joystick, etc. can be used to control the manipulator 16. By using the input apparatus 34 such as a joystick, the leading end position of the manipulator 16 can be moved on the order of micrometers, and the sample 40 can be manipulated with precision.

The force sensation presentation apparatus 36 is a means to manipulate the manipulator 16 and is configured to receive an input operation from the user for designating the position of the manipulator 16. The force sensation presentation apparatus 36 has a multi-joint arm 36a and is configured so that a leading end 36b of the multi-joint arm 36a can be moved in three axes of XYZ. The user performs an input operation for specifying the three-dimensional position of the manipulator 16 by gripping and moving the leading end 36b of the multi-joint arm 36a. The force sensation presentation apparatus 36 includes, for example, a sensor for identifying the position of the leading end 36b of the multi-joint arm 36a and transmits position information based on the position of the leading end 36b of the multi-joint arm 36a to the control apparatus 30.

The force sensation presentation apparatus 36 is also a force sensation presentation means for presenting a force applied to the manipulator 16 during cell manipulation to the user. The force sensation presentation apparatus 36 is configured so that a force sensation in three axes of XYZ can be presented at the leading end 36b of the multi-joint arm 36a. The force sensation presentation apparatus 36 has an actuator for applying a reaction force to the multi-joint arm 36a and controls the operation of the actuator based on force information transmitted from the control apparatus 30.

In the embodiment, the input apparatus 34 such as a joystick is used to manipulate the holding pipette 16a, and the force sensation presentation apparatus 36 is used to manipulate the injection pipette 16b. The force sensation presentation apparatus 36 may be used to manipulate the holding pipette 16a.

In the present disclosure, the three-dimensional positions of the sample 40 and the manipulator 16 are identified in real time, and the identified three-dimensional positions are used to estimate a force applied between the sample 40 and the manipulator 16. In this regard, a method for identifying the three-dimensional positions of the sample 40 and the manipulator 16 in real time will first be described.

(Three-dimensional position identification method) The control apparatus 30 identifies the three-dimensional position of the sample 40 included in the captured image based on the captured image captured by the imaging apparatus 24. The control apparatus 30 identifies the three-dimensional position of the sample 40 by using a first coordinate system with reference to the optical axis A of the objective lens 20. The control apparatus 30 identifies the sample 40 included in the captured image by using image recognition technology and identifies the position coordinates of the sample 40 in the x direction and the y direction from the central position of the sample 40 in the captured image. The control apparatus 30 identifies the position coordinate of the sample 40 in the z-direction based on the working distance identified when the sample 40 is imaged. The control apparatus 30 may, for example, define the leading end of the objective lens 20 to be the origin in the z direction (z=0) and the working distance from the objective lens 20 to the sample 40 as the position coordinate of the sample 40 in the z direction.

The working distance WD can be calculated based on the focal length $f_1$ of the objective lens 20, the focal length $f_2$ of the variable focus lens 22, the distance d between the objective lens 20 and the variable focus lens 22, and the refractive index distribution in the optical path from the objective lens 20 to the sample 40. The composite focal length $f_0$ of the combination of the objective lens 20 and the variable focus lens 22 is given by $f_0=f_1(f_2-d)/(f_1+f_2-d)$. The composite focal length $f_0$ corresponds to the working distance in the case where the optical path from the objective lens 20 to the sample 40 is air and the refractive index is approximately 1. In practice, the sample dish 46 and the liquid 48 are located in the optical path from the objective lens 20 to the sample 40. Therefore, the actual working distance WD will be different from the composite focal length $f_0$ due to the influence of the refractive index of the sample dish 46 and the liquid 48.

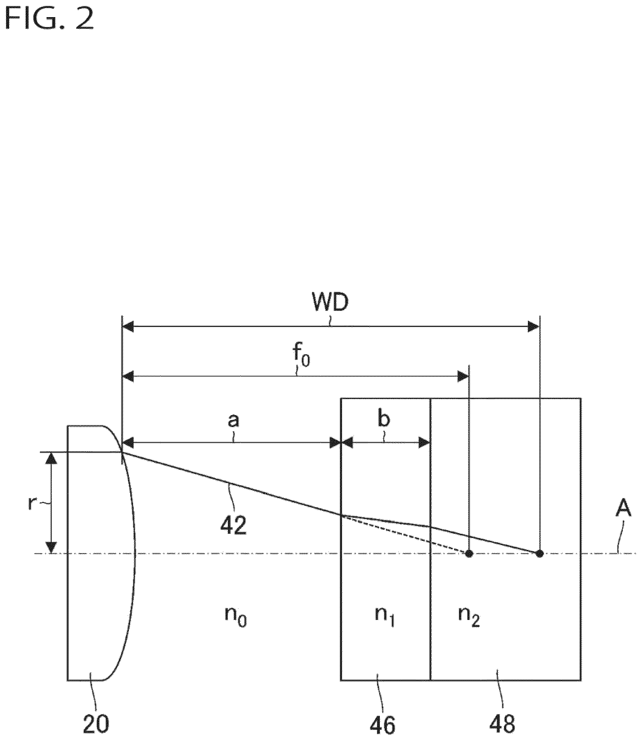

FIG. 2 is a diagram schematically showing the working distance WD identified when the influence of the refractive index is considered. As shown, the presence of the sample dish 46 and the liquid 48 in the optical path from the objective lens 20 to the sample 40 causes the observation light 42 incident on the objective lens 20 to be refracted. As a result, the actual working distance WD is longer than the composite focal length $f_0$ of the objective lens 20 and the variable focus lens 22 in the absence of the sample dish 46 and liquid 48. The actual working distance WD is given by the following expression (1) by using the synthetic focal length $f_0$, the refractive index $n_0$ of air, the refractive index $n_1$ of the sample dish 46, the refractive index $n_2$ of the liquid 48, the distance a from the objective lens 20 to the sample dish 46, the thickness b of the sample dish 46, and the effective radius r defined when the objective lens 20 is assumed to be an ideal plano-convex lens. Expression (1) can be derived based on a geometric relationship based on Snell's law.

$$\mathbf{WD} = a + b + \left(1 - \frac{a}{f_0} - \frac{n_0 b}{\sqrt{n_1^2 f_0^2 + (n_0^2 + n_1^2)r^2}}\right)\frac{\sqrt{n_2^2 f_0^2 + (n_0^2 + n_2^2)r^2}}{n_0} \quad \cdots \quad (1)$$

By way of one example of the actual working distance WD, WD=21.894 mm when the composite focal length $f_0$=21.059 mm, the refractive index of the polystyrene (PS) sample dish 46 is $n_1$=1.592, the refractive index of the pure water liquid 48 is $n_2$=1.33, the distance a=19.135 mm, the thickness b=1.0 mm, and the effective radius r=5.0 mm. In this case, the difference between the working distance WD and the composite focal length $f_0$ is 0.835 mm, which is very large compared to the depth of field (about 0.03 mm) of the objective lens 20 and the size of cells (about 0.1 mm). Therefore, the position coordinate of the sample 40 in the z direction can be accurately identified by correcting the working distance WD by considering the influence of the refractive index distribution from the objective lens 20 to the sample 40.

The control apparatus 30 controls the lens drive mechanism 28 to change the working distance WD. The control apparatus 30 identifies the position coordinate of the sample 40 in the z direction based on a plurality of images captured by varying the working distance WD. Specifically, the average edge intensity F of the sample 40 included in each of the plurality of captured images is calculated, and the position coordinate of the sample 40 in the z direction is identified based on the maximum value of the intensity found when the correlation between the working distance WD and the average edge intensity F is approximated by a Gaussian distribution. The average edge intensity F is determined by calculating the edge intensity f (x, y) of each pixel in the captured image and averaging the edge intensity f (x, y) of all pixels in an area including the sample 40. Denoting the luminance value of each pixel by I(x,y), the edge intensity is given by $f(x,y)=\{(I(x+1,y)-I(x,y))^2+(I(x,y+1)-I(x,y))^2\}^{1/2}$. The average edge intensity F indicates the contrast of the sample 40 included in the captured image, and the larger the average edge intensity F, the more in focus a high-contrast image of the sample 40 is captured.

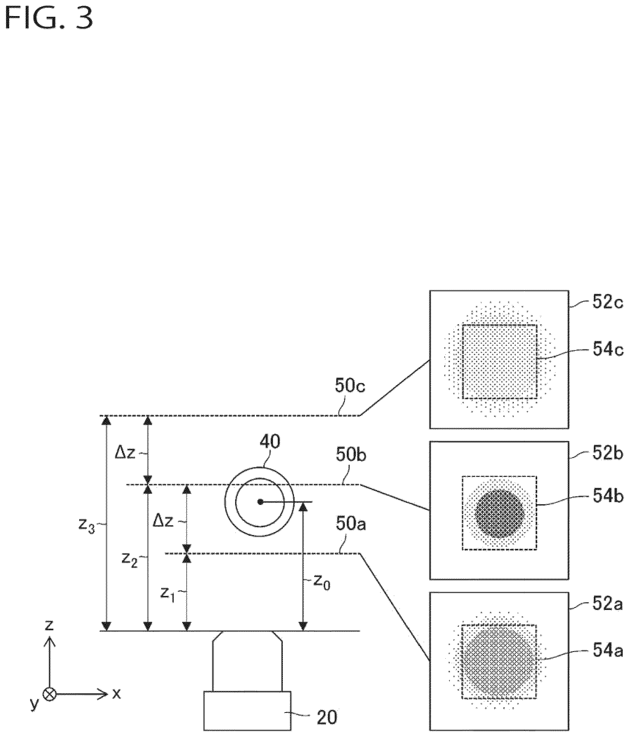

FIG. 3 is a diagram schematically showing a method for identifying the position coordinate of the sample 40 in the z direction. FIG. 3 schematically shows images 52a, 52b, and 52c obtained when the working distance WD of the objective lens 20 is set to the first distance $z_1$, the second distance $z_2$, and the third distance $z_3(z_1<z_2<z_3)$ and capturing focal planes 50a, 50b, and 50c located at the respective distances $z_1$-$z_3$. The illustrated example shows a state in which the central coordinate z of the sample 40 is located between the first distance $z_1$ and the second distance $z_2$, and the sample 40 intersects the second focal plane 50b. Since the second focal plane 50b intersects the sample 40, the second captured image 52b obtained by imaging the second focal plane 50b includes the sample 40 in focus. On the other hand, the first captured image 52a obtained by imaging the first focal plane 50a away from the sample 40 includes the sample 40 in a blurred focus. Further, the third captured image 52c obtained by imaging the third focal plane 50c farther away from the sample 40 includes the sample 40 out of focus. The interval Δz between the first distance $z_1$, the second distance $z_2$, and the third distance $z_3$ is set to a value about 1-2 times the size of the sample 40. When the size of the sample 40 is about 100 μm, for example, Δz=100 μm-200 um approximately.

The control apparatus 30 acquires the plurality of captured images 52a-52c captured by changing the working distance WD and calculates the average edge intensity $F_1$, $F_2$, and $F_3$ in areas 54a-54c including the sample 40 in the respective captured images 52a-52c. In the example shown in FIG. 3, the relative magnitude of the average edge intensity of the captured images 52a-52c is $F_3<F1<F_2$. The control apparatus 30 approximates a correlation between the calculated average edge intensities $F_1$, $F_2$, and $F_3$ and the distances $z_1$, $z_2$, and $z_3$ as far as the respective focal planes 50a-50c by a Gaussian distribution.

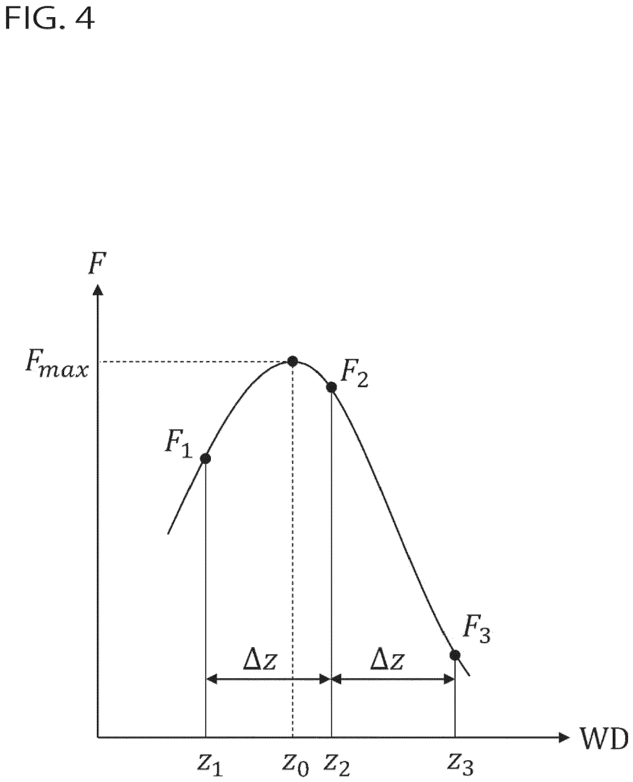

FIG. 4 is a graph showing a relationship between the average edge intensities $F_1$, $F_2$, and $F_3$ and the working distance WD approximated by a Gaussian distribution. As illustrated, the average edge intensities $F_1$-$F_3$ can be approximated by a Gaussian distribution to identify the maximum value $F_{max}$, provided that the relative magnitude of the average edge intensity is such that $F_1<F_2$ and $F_3<F_2$. Since the maximum value $F_{max}$ corresponds to the position where the sample 40 is most in focus, the working distance z0 corresponding to the maximum value $F_{max}$ can be regarded as the z-coordinate of the center of the sample 40. The working distance z0 corresponding to the maximum value F max can be calculated according to the following expression (2) by using the average edge intensities $F_1$, $F_2$, and $F_3$ and the distances $z_1$, $z_2$, and $z_3$ to the respective focal planes.

$$z_0 = \frac{(\ln F_2 - \ln F_3)(z_2^2 - z_1^2) - (\ln F_2 - \ln F_1)(z_2^2 - z_3^2)}{2\Delta z\{(\ln F_2 - \ln F_1) + (\ln F_2 - \ln F_3)\}} \quad \cdots (2)$$

The control apparatus 30 identifies the three-dimensional position of the manipulator 16 based on the operation of the manipulator drive mechanism 26. First, the control apparatus 30 identifies the three-dimensional position of the manipulator 16 by using the second coordinate system with reference to the drive axis of the manipulator 16. The three-dimensional position of the manipulator 16 with reference to the second coordinate system can be relatively identified by calculating the amount of movement of the manipulator 16 in the three-dimensional direction from the rotation angle of the motor of the manipulator drive mechanism 26, the amount of driving the actuator, etc. The second coordinate system is set, for example, for each of the holding pipette 16a and the injection pipette 16b and, in each second coordinate system, the three-dimensional positions of leading ends 38a and 38b of the holding pipette 16a and the injection pipette 16b are identified. The second coordinate system may be a coordinate system common to the holding pipette 16a and the injection pipette 16b.

The control apparatus 30 identifies the three-dimensional position of the manipulator 16 in the first coordinate system by performing coordinate conversion from the second coordinate system to the first coordinate system. As described above, the first coordinate system is a coordinate system with reference to the optical axis A of the objective lens 20. Given position coordinates $P_1(X_1,Y_1,Z_1)$ in the first coordinate system and position coordinates $P_2(X_2,Y_2,Z_2)$ in the second coordinate system, coordinate conversion from the second coordinate system to the first coordinate system can be given by the following expression (3).

$$\begin{bmatrix} X_1 \\ Y_1 \\ Z_1 \end{bmatrix} = \begin{bmatrix} r_{11} & r_{12} & r_{13} & t_1 \\ r_{21} & r_{22} & r_{23} & t_2 \\ r_{31} & r_{32} & r_{33} & t_3 \end{bmatrix} \begin{bmatrix} X_2 \\ Y_2 \\ Z_2 \\ 1 \end{bmatrix} \quad \cdots \quad (3)$$

In the above expression (3), $r_{ij}$ is a conversion parameter indicating rotation from the second coordinate system to the first coordinate system, and $t_i$ is a conversion parameter indicating a translation from the origin in the second coordinate system to the origin in the first coordinate system. The coordinate conversion parameters $r_{ij}$ and $t_i$ can be derived from the correlation between the position coordinates of the leading end of the manipulator 16 measured in the second coordinate system and the position coordinates of the leading end of the manipulator 16 measured in the first coordinate system. Specifically, the leading end of the manipulator 16 is arranged at a plurality of (about 100-200) different measurement positions, and the position coordinates of the leading end of the manipulator 16 at each measurement position are measured in each of the first and second coordinate systems. Subsequently, the coordinate transformation parameters $r_{ij}$ and $t_i$ can be estimated by identifying the correlation between position coordinates in the first and second coordinate systems by using the least squares method.

The position coordinates in the first coordinate system can be identified from the working distance WD of the objective lens 20 found when the leading end of the manipulator 16 is imaged and from the coordinates (u, v) of the pixel at the leading end of the manipulator 16 in the captured image. The relationship between the position coordinates $P_1(X_1, Y_1, Z_1)$ in the first coordinate system and the coordinates (u, v) of the pixel in the captured image can be represented by the following expression (4) by perspective projection transform.

$$s \begin{bmatrix} u \\ v \\ 1 \end{bmatrix} = \begin{bmatrix} WD & 0 & c_x \\ 0 & WD & c_y \\ 0 & 0 & 1 \end{bmatrix} \begin{bmatrix} X_1 \\ Y_1 \\ Z_1 \\ 1 \end{bmatrix} \quad \cdots \quad (4)$$

In expression (4), $(c_x, c_y)$ denotes the central coordinates of the captured image, and s denotes an arbitrary constant. Satisfying both expression (3) and expression (4), the following expression (5) is obtained.

$$s \begin{bmatrix} u \\ v \\ 1 \end{bmatrix} = \begin{bmatrix} WD & 0 & c_x \\ 0 & WD & c_y \\ 0 & 0 & 1 \end{bmatrix} \begin{bmatrix} r_{11} & r_{12} & r_{13} & t_1 \\ r_{21} & r_{22} & r_{23} & t_2 \\ r_{31} & r_{32} & r_{33} & t_3 \end{bmatrix} \begin{bmatrix} X_2 \\ Y_2 \\ Z_2 \\ 1 \end{bmatrix} \quad \cdots \quad (5)$$

By using expression (5), the coordinate conversion parameters $r_{ij}$ and $t_i$ can be estimated based on the working distance WD of the objective lens 20, the coordinates (u, v) of the pixel in the captured image, and the position coordinates $P_2(X_2, Y_2, Z_2)$ in the second coordinate system.

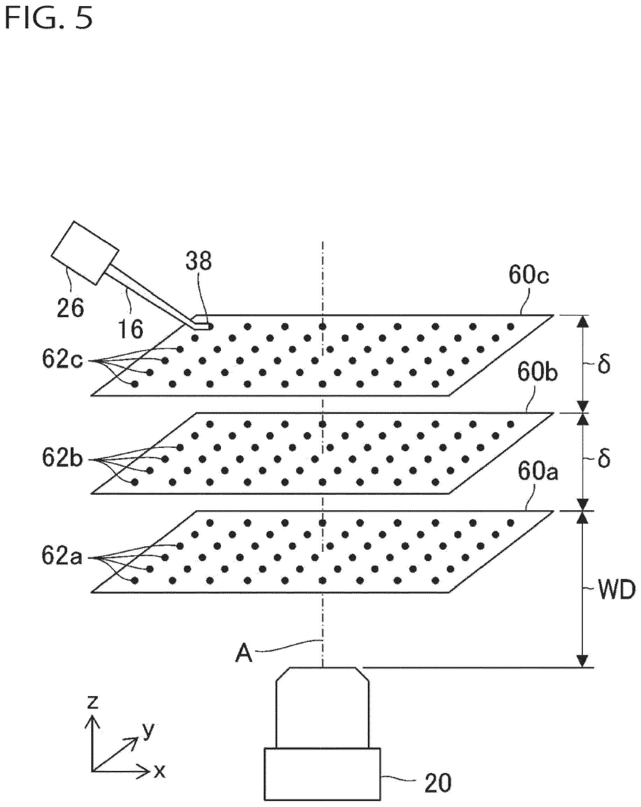

FIG. 5 is a diagram schematically showing a measurement pattern for the manipulator 16 for deriving coordinate conversion parameters. In the first coordinate system with reference to the optical axis A of the objective lens 20, a plurality of measurement planes 60a, 60b, and 60c spaced apart by a distance δ in the z direction are set, and a plurality of measurement positions 62a, 62b, and 62c are set in a grid pattern in the respective measurement planes 60a-60c. In the illustrated example, three measurement planes 60a-60c are set, and 9×6=54 measurement positions (e.g., the plurality of first measurement positions 62a) are set in one measurement plane (e.g., the first measurement plane 60a). The number of measurement planes and the number of measurement positions are not limited to the numbers described above. The number of measurement planes may be 4 or more, and the number of measurement positions in each measurement plane may be more than or less than 54.

First, the manipulator 16 is moved so that the leading end 38 of the manipulator 16 is located within the field of view of the objective lens 20. The working distance WD of the objective lens 20 is then adjusted so that the leading end 38 of the manipulator 16 is in focus. Thereby, the distance WD to the first measurement plane 60a is determined. Subsequently, the manipulator 16 is moved while the leading end 38 of the manipulator 16 is in focus, and the position coordinates of the leading end 38 of the manipulator 16 are identified in each of the first and second coordinate systems at the plurality of measurement positions 62a in the first measurement plane 60a.

When measurement at all measurement positions 62a in the first measurement plane 60a is completed, the leading end 38 of the manipulator 16 is then moved to the second measurement plane 60b. In this process, the leading end 38 of the manipulator 16 is moved by $\delta$ in the z direction, and the working distance is increased by $\delta$ to WD+$\delta$. The distance $\delta$ between the first measurement plane 60a and the second measurement plane 60b is set to a value larger than the depth of field of the objective lens 20 and is, for example, about 5 times-10 times the depth of field. By way of example, the depth of field of the objective lens 20 is 30 $\mu$m, and $\delta$=200 um. Therefore, the plurality of measurement positions 62a, 62b, and 62c in the measurement pattern of FIG. 5 are distributed over a range wider than the depth of field of the objective lens 20 with respect to the optical axis direction (z direction) of the objective lens 20.

In the second measurement plane 60b, as in the first measurement plane 60a, the manipulator 16 is moved while the leading end 38 of the manipulator 16 is in focus. Thereby, the position coordinates of the leading end 38 of the manipulator 16 are identified in each of the first and second coordinate systems at the plurality of measurement positions 62b in the second measurement plane 60b. When measurement at all measurement positions 62b in the second measurement plane 60b is completed, the manipulator 16 is moved by $\delta$ in the z direction so that the leading end 38 of the manipulator 16 is located on the third measurement plane 60c, and the working distance WD is increased by $\delta$ to WD+2$\delta$. Subsequently, the position coordinates of the leading end 38 of the manipulator 16 are identified in each of the first and second coordinate systems at the plurality of measurement positions 62c in the third measurement plane 60c.

After identifying the position coordinates at all measurement positions 62a, 62b, and 62c, the control apparatus 30 determines the coordinate conversion parameters $r_{ij}$ and $t_i$ by using the above expression (5). The control apparatus 30 may individually measure the leading ends 38a and 38b of the holding pipette 16a and the injection pipette 16b to determine the coordinate conversion parameters $r_{ij}$ and $t_i$. Once the coordinate conversion parameters $r_{ij}$ and $t_i$ are determined, they can be used continuously as long as the apparatus configuration remains unchanged. That is, the control apparatus 30 need not determine the coordinate conversion parameters $r_{ij}$ and $t_i$ for each use of the manipulation system 10. The control apparatus 30 may store coordinate conversion parameters $r_{ij}$ and $t_i$ determined in advance and use the coordinate conversion parameters $r_{ij}$ and $t_i$ thus stored to convert the position coordinates of the leading end 38 of the manipulator 16.

The coordinate conversion parameters $r_{ij}$ and $t_i$ convert the relative movement of the manipulator 16 in the second coordinate system to the relative movement in the first coordinate system with precision. On the other hand, our knowledge shows that, if a coordinate point in the second coordinate system is converted to a coordinate point in the first coordinate system by using the coordinate conversion parameters $r_{ij}$ and $t_i$, the coordinate points are misaligned. That is, the coordinate conversion parameters $r_{ij}$ and $t_i$ used alone result in poor precision of absolute values of position coordinates. The above-described coordinate conversion parameters $r_{ij}$ and $t_i$ are determined by using the property of perspective projection transform that gives an appearance such that the closer an imaged object is to the viewpoint, the larger it appears, and the farther it is, the smaller it appears. In the case of a normal camera, the depth of field of the camera is relatively large so that the apparent size of an object can be made to vary significantly, by shifting the position of the object in the depth direction within the depth of field of the camera. In the case of a microscope, on the other hand, the depth of field of the objective lens 20 is small so that the apparent size of an object does not change substantially even if the position of the object in the depth direction is shifted within the depth of field of the objective lens 20. That is, the apparent change in a captured image is small when the coordinate of the leading end 38 of the manipulator 16 in the z direction is changed. If the coordinate in the z direction of the first coordinate system is estimated based on the captured image, deviation from the actual position (true value) will be large. In the present disclosure, therefore, as in the case of the method for identifying the position coordinate of the sample 40 in the z direction, the absolute value of the z coordinate of the leading end 38 of the manipulator 16 is identified based on the average edge intensity F in the captured image capturing the leading end 38 of the manipulator 16.

Hereinafter, a method for calibrating the origin of the manipulator 16 will be described. The control apparatus 30 first positions the leading end 38 of the manipulator 16 at an initial point (tentative origin) O set in the field of view of the objective lens 20. The control apparatus 30 calculates the position coordinates $(X_{10}, Y_{10}, Z_{10})$ of the initial point O in the first coordinate system by subjecting the position coordinates $(X_{20}, Y_{20}, Z_{20})$ of the initial point O in the second coordinate system to coordinate conversion. The control apparatus 30 captures an image of the leading end 38 of the manipulator 16 located at the initial point O while changing the working distance WD to generate a plurality of captured images. The change rate of the working distance WD is preferably as small as possible and is preferably equal to or less than the depth of field of the objective lens 20 (e.g., about 1 pm) . The control apparatus 30 calculates the average edge intensity F of the leading end 38 of the manipulator 16 included in each of the plurality of captured images and identifies a captured image in which the average edge intensity F is the maximum. The control apparatus 30 defines the working distance $WD_{max}$ found when the captured image with the maximum average edge intensity F is captured to be the true value of the z coordinate of the initial point O. Given that the pre-calibration z coordinate of the leading end 38 of the manipulator 16 in the first coordinate system is Z1 and the post-calibration z coordinate is $Z_1{'}$, $Z_1{'} = Z_1 - Z_{10} + WD_{max}$.

While the manipulation system 10 is being used, the control apparatus 30 identifies the three-dimensional position of the sample 40 in the first coordinate system within the field of view of the objective lens 20 in real time and identifies the three-dimensional position of the leading end 38 of the manipulator 16 in the first coordinate system in real time. The three-dimensional position of the sample 40 can be identified by changing the working distance WD to acquire at least three captured images. The time for identifying the three-dimensional position of the sample 40 is mainly determined by the time required to drive the variable focus lens 22 to change the working distance WD. The time required to change the working distance WD depends on the specification of the variable focus lens 22 and the amount of change in the working distance WD. By way of one example, the time is about 10 milliseconds-20 milliseconds. Therefore, the time required to acquire three captured images by changing the working distance WD is about 30 milliseconds-60 milliseconds, and the three-dimensional position of the sample 40 can be captured at a cycle of 16-33 times per second. Meanwhile, the three-dimensional position of the leading end 38 of the manipulator 16 can be successively acquired based on the operation of the manipulator drive mechanism 26 and can be identified within the time for identifying the three-dimensional position of the sample 40.

A description will now be given of a method for presenting the three-dimensional positions of the sample 40 and the manipulator 16.

(Three-dimensional position presentation method)

The control apparatus 30 maps the three-dimensional relative arrangement of the sample 40 and the manipulator 16 on a virtual space based on the three-dimensional positions of the sample 40 and the manipulator 16 identified. The range of the virtual space to map to is wider than the range of the field of view of the objective lens 20. The three-dimensional position of the sample 40 can be identified only within the field of view of the objective lens 20, but the three-dimensional position of the manipulator 16 can be identified both within and outside the field of view of the objective lens 20. In the virtual space, therefore, the position of the leading end 38 of the manipulator 16 can be mapped at all times even if the leading end 38 of the manipulator 16 is not located in the field of view of the objective lens 20. The three-dimensional relative arrangement of the sample 40 and the manipulator 16 mapped to the virtual space is displayed on the three-dimensional display apparatus 32a in real time. The display cycle of the three-dimensional display apparatus 32a is 40 milliseconds (25 frames per second) by way of example.

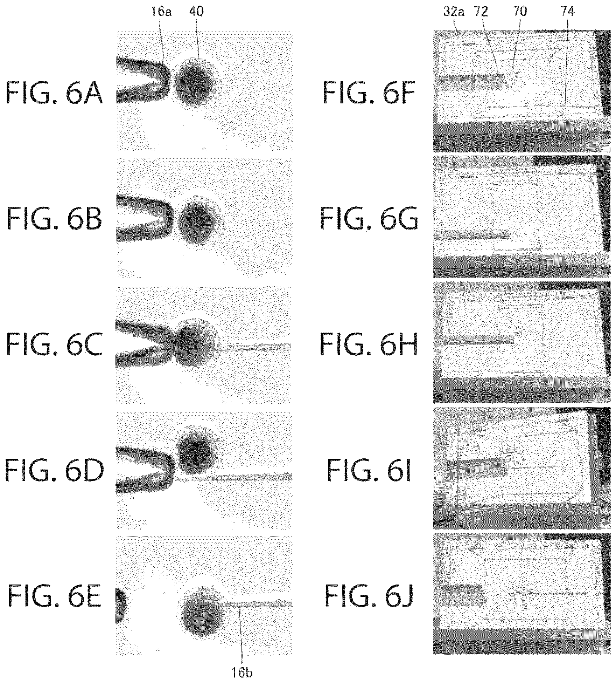

FIGS. 6A-6J are diagrams showing images captured in the field of view of the objective lens 20 and examples displayed on the three-dimensional display apparatus 32a. The figures show a flow in which the sample 40 is fixed with the holding pipette 16a from the left side and the sample 40 is manipulated by the injection pipette 16b from the right side in a time series. FIGS. 6A-6E show images captured within the field of view of the objective lens 20 (i.e., microscopic images), and FIGS. 6F-6J show examples displayed on the three-dimensional display apparatus 32a corresponding to FIGS. 6A-6E, respectively. The three-dimensional display apparatus 32a displays a first object 70 simulating the sample 40, a second object 72 simulating the holding pipette 16a, and a third object 74 simulating the injection pipette 16b in three dimensions.

FIGS. 6A and 6B show a state that occurs before the injection pipette 16b is brought closer to the sample 40, and the injection pipette 16b is not shown in the captured image. However, the three-dimensional position of the injection pipette 16b can be captured even if it is outside the field of view of the objective lens 20. Therefore, the three-dimensional display apparatus 32a in FIGS. 6F and 6G equally displays the third object 74 simulating the injection pipette 16b. As a result, a user can manipulate the injection pipette 16b to make the leading end of the injection pipette 16b approach the sample 40 while viewing the three-dimensional display apparatus 32a even if the injection pipette 16b is not visible in the field of view of the objective lens 20.

The three-dimensional display apparatus 32a can arbitrarily switch the viewpoint. For example, the three-dimensional display apparatus 32a can switch to a top view obtained when the sample 40 is viewed in the z direction, a side view obtained when the sample 40 is viewed in the x direction, and a side view obtained when the sample 40 is viewed in the y direction. FIG. 6F is an example of a top view displayed when the sample 40 is viewed in the z direction, and FIG. 6G is an example of a side view displayed when the sample 40 is viewed in the y direction. By switching the viewpoint appropriately, the insertion angle of the manipulator 16 with respect to the sample 40 can be easily checked.

FIGS. 6C and 6D show a state in which the injection pipette 16b is brought into contact with the sample 40 for manipulation. Referring to the captured image of FIG. 6C, it appears that the injection pipette 16b is piercing the sample 40. Referring to the three-dimensional display apparatus 32a of FIG. 6H, however, it can be seen that the injection pipette 16b is under the sample 40, and the injection pipette 16b is not piercing the sample 40. Further, it can be seen that the sample 40 is lifted upward by the injection pipette 16b, and the sample 40 is about to come off from the holding pipette 16a.

FIG. 6E shows a state in which the injection pipette 16b is piercing the sample 40, and the sample 40 is isolated from the holding pipette 16a. In this instance, it is possible to confirm how deep the injection pipette 16b is piercing the sample 40 by viewing the three-dimensional display apparatus 32a of FIG. 6I. Further, it is possible to cheek whether the injection pipette 16b is piercing the center of the sample 40 near the center by switching the viewpoint of the three-dimensional display apparatus 32a.

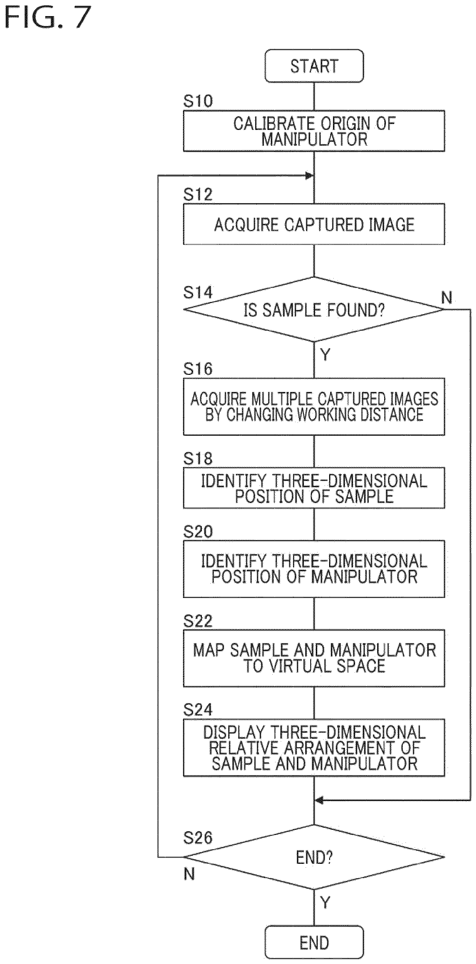

FIG. 7 is a flowchart showing a three-dimensional position presentation method according to the embodiment. First, the origin of the manipulator 16 is calibrated (S10). Calibration of the origin of the manipulator 16 is performed by imaging the leading end 38 of the manipulator 16 at different working distances and identifying the working distance $WD_{max}$ at which the average edge intensity F of the captured image is maximized. Subsequently, a captured image obtained by imaging an observation area is acquired (S12). When the sample 40 is located in the captured image (Y in S14), a plurality of captured images are acquired by changing the working distance WD (S16). The three-dimensional position of the sample 40 is identified based on the maximum value found when the average edge intensity F of the sample 40 included in the plurality of captured images is approximated by a Gaussian distribution (S18). Subsequently, the three-dimensional position of the manipulator 16 in the second coordinate system is identified based on the operation of the manipulator 16, and the three-dimensional position of the manipulator 16 in the first coordinate system is identified by coordinate conversion from the second coordinate system to the first coordinate system (S20). The sample 40 and the manipulator 16 are mapped to the virtual space (S22) based on the three-dimensional positions of the sample 40 and the manipulator 16 in the identified first coordinate system, and the three-dimensional relative arrangement of the sample 40 and the manipulator 16 mapped to the virtual space is displayed on the three-dimensional display apparatus 32a, etc. (S24). When the sample 40 is not found in S14 (N in S14), the steps of S16-S24 are skipped. When the manipulation system 10 continues to be used (N in S26), the steps of S12-S24 are repeated. When the use of the manipulation system 10 is terminated (Y in S26), the flow is terminated.

According to an example of this disclosure, the steps of S12-S24 are repeated every 40 milliseconds, and the three-dimensional positions of the sample 40 and the manipulator 16 are updated and displayed at a cycle of 25 frames per second. Since this display cycle is almost the same as the frame rate of common moving images, the three-dimensional positions of the sample 40 and the manipulator 16 appear to be immediately reflected without any time lag to the user viewing the three-dimensional display apparatus 32a. As a result, the user can accurately manipulate the sample 40 with the manipulator 16 while viewing the three-dimensional display apparatus 32a without feeling stress due to misalignment between the actual position and the displayed position. Since the three-dimensional display apparatus 32a presents the relative arrangement of the sample 40 and the manipulator 16 such that stereoscopic vision is enabled, the user can check the relative arrangement of the sample 40 and the manipulator 16 at various angles by changing the direction of the line of sight relative to the three-dimensional display apparatus 32a. In other words, since the sample 40 and the manipulator 16 can be displayed on an enlarged scale as if they exist in front of the eyes three-dimensionally, it is easy to grasp the three-dimensional relative positions of the two. This improves the operability experienced when the user manipulates the minute sample 40 such as a cell with precision and enhances the convenience of the manipulation system 10.

A method for estimating a force applied between the sample 40 and the manipulator 16 will then be described.

(Force estimation method)

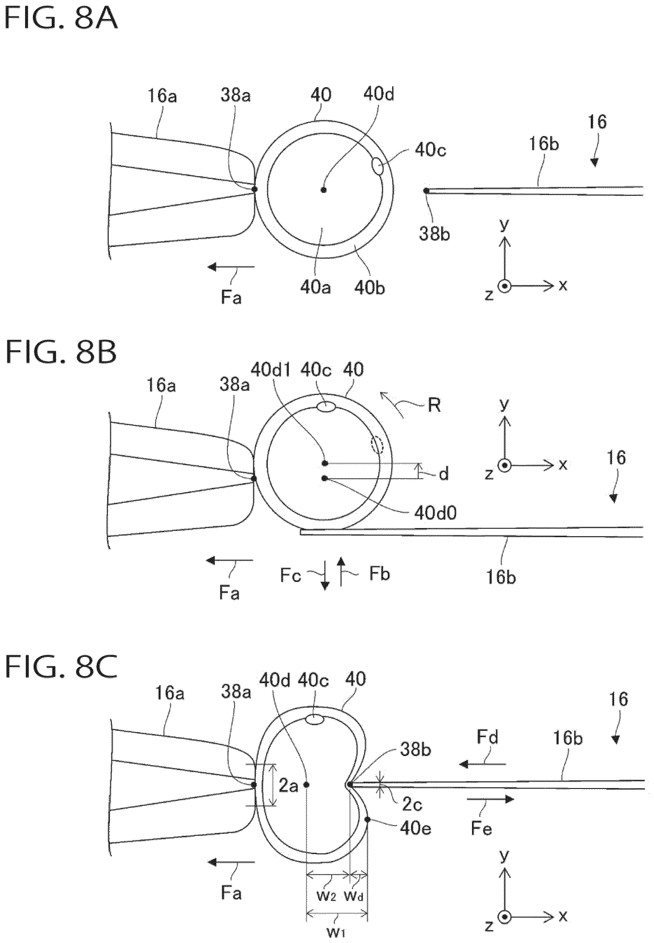

FIGS. 8A-8C are diagrams schematically showing how the sample 40 is manipulated by the manipulator 16. FIG. 8A shows an operation of holding the sample 40, FIG. 8B shows an operation of rotating the sample 40, and FIG. 8C shows an operation of piercing the sample 40. FIGS. 8A-8C correspond to images captured by the imaging apparatus 24 and show states in which the sample 40 is viewed in the z direction. In FIGS. 8A-8C, the direction of protrusion of the leading end 38a of the holding pipette 16a is shown as the +x direction.

FIG. 8A shows an operation of holding the sample 40 by using the holding pipette 16a. The sample 40 is fixed by bringing the leading end 38a of the holding pipette 16a in close proximity to the sample 40 and sucking the sample 40 by the leading end 38a of the holding pipette 16a. The sample 40 is in contact with the leading end 38a of the holding pipette 16a in the x direction, and a holding force Fa in the x direction (-x direction) caused by suction is applied to the sample 40. An example of the sample 40 is a mature human or animal egg cell having a central cytoplasm 40a, a zona pellucida 40b surrounding the cytoplasm 40a, and a polar body 40c at a certain location between the cytoplasm 40a and zona pellucida 40b.

FIG. 8B illustrates an operation of rotating the sample 40 by using the injection pipette 16b. Referring to FIG. 8B, the position of the polar body 40c is adjusted by rotating the sample 40 as indicated by the arrow R. For example, in the piercing operation of FIG. 8C, the orientation of the sample 40 is adjusted so that the polar body 40c is located in the y direction from a center 40d of the sample 40 in order to prevent the spindle located in the vicinity of the polar body 40c from being damaged by the injection pipette 16b.

In the rotation operation of FIG. 8B, the injection pipette 16b is moved in a direction perpendicular to the direction of protrusion (e.g., the y direction) instead of the direction of protrusion (e.g., the x direction) of the leading end 38b of the injection pipette 16b. By pressing the side of the injection pipette 16b against the sample 40, the position and orientation of the sample 40 are shifted. When the injection pipette 16b is pressed against the sample 40 in the +y direction, for example, a force Fb in the +y direction is applied to the sample 40, and the central position of the sample 40 is shifted from 40d0 to 40d1 in the +y direction. In this process, the sample 40 will return to its original position due to the holding force Fa exerted by the holding pipette 16a so that a reaction force Fc caused by the holding force Fa is applied to the injection pipette 16b. The direction of this reaction force Fc is opposite to the displacement d from the pre-manipulation central position 40d0 of the sample 40 to the post-manipulation central position 40d1 of the sample 40. Further, the magnitude of the reaction force Fc is correlated with the magnitude of displacement (i.e., the amount of displacement) of the sample 40 caused by the manipulation by using the injection pipette 16b. The reaction force Fc can be given by, for example, the following expression (6).

$$\vec{F_c} = -f\left(|\vec{d}|\right)\frac{\vec{d}}{|\vec{d}|} \quad \cdots (6)$$

where the function f is a function showing a correlation between the magnitude of the reaction force Fc and the magnitude of the displacement d of the sample 40. The function f may have the magnitude of the holding force Fa as a variable and may be represented as f (d, Fa). Assuming that the magnitude of the reaction force Fc and the magnitude of the displacement d of the sample 40 are proportional, expression (6) can be represented by the following expression (7) by using a proportionality constant k.

$$\vec{F_c} = -k \cdot \vec{d} \quad \cdots (7)$$

The amount of displacement d in expressions (6) and (7) above can be identified in real time based on a change in the position of the center 40d of the sample 40. The control apparatus 30 identifies the amount of displacement d at each moment by identifying the position of the center 40d of the sample 40 by using the above-described three-dimensional position identification method based on a captured image. The control apparatus 30 estimates the force Fc applied between the sample 40 and the injection pipette 16b at each moment based on the identified amount of displacement d.

FIG. 8C illustrates an operation of inserting the injection pipette 16b into the sample 40. Referring to FIG. 8C, a force Fd in the -x direction is applied to the sample 40 by pressing the leading end 38b of the injection pipette 16b against the sample 40 in the -x direction. The sample 40 is sandwiched between the holding pipette 16a and the injection pipette 16b and is deformed accordingly, and the point where the sample 40 comes into contact with the leading end 38b of the injection pipette 16b becomes concave in the -x direction. In this process, a reaction force Fe caused by the elastic force that causes the sample 40 to return to its original shape is applied to the injection pipette 16b. The direction of this reaction force Fe is opposite (+x direction) to the direction of protrusion (-x direction) of the leading end 38b of the injection pipette 16b. Further, the magnitude of the reaction force Fe is correlated with the magnitude of the amount of deformation wd of the sample 40, and can be given by, for example, the following expression (8).

$$\vec{F_e} = -\frac{2\pi E h w_d^3}{a^2(1-v)}\left[\frac{3 - 4\zeta^2 + \zeta^4 + 2\ln\zeta^2}{(1-\zeta^2)\left(1-\zeta^2+\ln\zeta^2\right)^3}\right] \cdot \vec{e} \quad \cdots (8)$$

where E denotes the elastic modulus (Young's modulus) of the sample 40, v denotes the Poisson's ratio of the sample 40, and h denotes the thickness of the zona pellucida. Further, $\zeta = c/a$, a denote the radius of the leading end 38a of the holding pipette 16a, and c denotes the radius of the leading end 38b of the injection pipette 16b. Further, the vector e denotes the direction of protrusion of the injection pipette 16b. Details of expression (8) are described in "Y. Sun, K. T. Wan, K. P. Roberts, J. C. Bischof and B. J. Nelson: "Mechanical property characterization of mouse zona pellucida", IEEE Trans Nanobioscience, vol. 2, no. 4, pp. 279.286, 2003."

The amount of deformation wd in expression (8) above corresponds to the amount of concavity in the sample 40 in the direction of protrusion (x direction) of the leading end 38b of the injection pipette 16b and corresponds to the distance in the direction of protrusion (x direction) from an end 40e of the sample 40 in the direction of protrusion (x direction) to the leading end 38b of the injection pipette 16b. The amount of deformation wd can be measured based on, for example, the captured image of the sample 40. The amount of deformation wd may be identified as a difference between a distance w1 from the center 40d to the end 40e of the sample 40 in the X direction and a distance w2 from the center 40d of the sample 40 to the leading end 38b of the injection pipette 16b in the x direction (i.e., wd=w1-w2). In this case, the positions of the center 40d and the end 40e of the sample 40 can be identified based on the captured image of the sample 40. The position of the leading end 38b of the injection pipette 16b can be identified by a method using coordinate conversion in the three-dimensional position identification method described above.

The control apparatus 30 identifies the position of the center 40d of the sample 40 in real time by using the above-described three-dimensional position identification method based on a captured image. The control apparatus 30 identifies the positions of the center 40d and the end 40e of the sample 40 in real time based on the captured image that is most focused on the sample 40 and identifies the distance w1. The control apparatus 30 identifies the position of the leading end 38b of the injection pipette 16b in real time by coordinate conversion of the three-dimensional position with reference to the drive axis of the injection pipette 16b. Thereby, the control apparatus 30 identifies the amount of deformation wd at each moment. The control apparatus 30 estimates the force Fe applied between the sample 40 and the injection pipette 16b at each moment based on the identified amount of deformation wd.

The control apparatus 30 transmits force information based on the estimated forces Fc and Fe to the force sensation presentation apparatus 36 to cause the force sensation corresponding to the estimated forces Fc and Fe to be presented to the user through the force sensation presentation apparatus 36. Since the magnitude of the estimated forces Fc and Fe is very small and is about 1 $\mu$N-1 mN, it is difficult for the user to perceive the slight force if the magnitude of the estimated forces Fc and Fe is transmitted to the force sensation presentation apparatus 36 as they are. In the present disclosure, therefore, the estimated force is amplified before being presented to the user. The control apparatus 30 generates force information indicating forces $\alpha$Fc and $\alpha$Fe obtained by multiplying the estimated forces Fc and Fe by a predetermined amplification factor $\alpha$ and transmits the force information to the force sensation presentation apparatus 36. The amplification factor $\alpha$ is 100 times or more and 10000 times or less and is, for example, 1000 times or more and 5000 times or less. The force sensation presentation apparatus 36 presents the amplified forces $\alpha$Fc and $\alpha$Fe by activating the actuator based on the force information acquired from the control apparatus 30. Given that the amplification factor $\alpha$ is 1000 times, for example, a force sensation of about 1 mN-1 N is presented so that the user can easily perceive the reaction force associated with the manipulation by the manipulator 16. The amplification factor $\alpha$ may be a parameter that can be appropriately adjusted by the user.

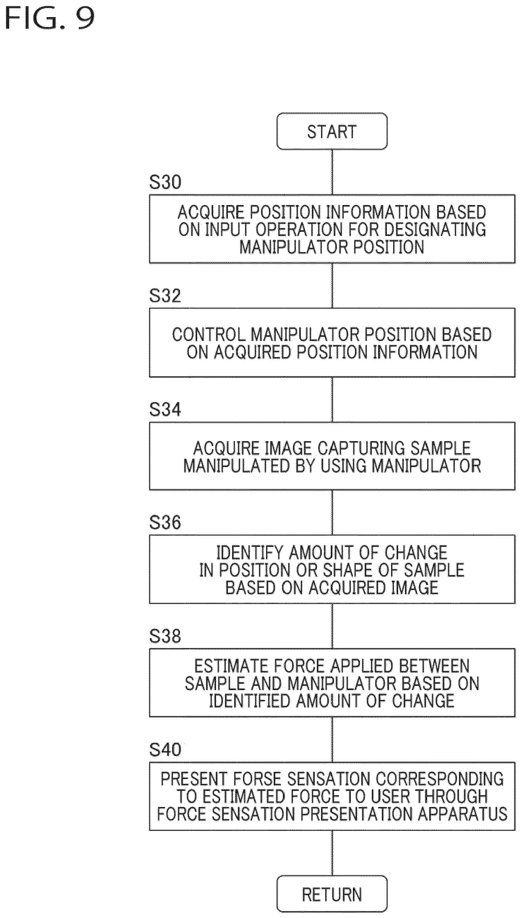

FIG. 9 is a flowchart showing a force sensation presentation method according to the embodiment. The control apparatus 30 acquires position information based on the user's input operation for designating the position of the manipulator 16 from the force sensation presentation apparatus 36 (S30). For example, position information corresponding to the position of the leading end 36b of the multi-joint arm 36a is transmitted to the control apparatus 30 in response to the user operation of gripping and moving the leading end 36b of the multi-joint arm 36a. Subsequently, the control apparatus 30 controls the operation of the manipulator drive mechanism 26 based on the acquired position information and controls the position of the manipulator 16 (S32). The imaging apparatus 24 captures an image of the sample 40 manipulated by using the manipulator 16 through the objective lens 20, and the control apparatus 30 acquires the image captured by the imaging apparatus 24 (S34) .

Subsequently, the control apparatus 30 identifies an amount of change in the position or shape of the sample 40 based on the acquired image (S36) and estimates the force applied between the sample 40 and the manipulator 16 based on the identified amount of change (S38). When the rotation operation shown in FIG. 8B is performed, for example, the control apparatus 30 identifies the amount of displacement d at the central position of the sample 40 and estimates the direction and magnitude of the reaction force Fc applied to the injection pipette 16b based on the identified amount of displacement d. When the piercing operation shown in FIG. 8C is performed, the control apparatus 30 identifies the amount of deformation wd of the sample 40 and estimates the direction and magnitude of the reaction force Fe applied to the injection pipette 16b based on the identified amount of deformation wd. The control apparatus 30 transmits force information corresponding to the estimated forces Fc and Fe to the force sensation presentation apparatus 36 to cause the force sensation to be presented to the user through the force sensation presentation apparatus 36 (S40). For example, the control apparatus 30 transmits force information indicating the forces $\alpha$Fc and $\alpha$Fe obtained by amplifying the estimated forces Fc and Fe by the predetermined amplification factor $\alpha$ to the force sensation presentation apparatus 36 to cause the amplified force to be presented to the user.

According to the present disclosure, the user can manipulate the manipulator 16 by using the force sensation presentation apparatus 36 while feeling the reaction force applied to the manipulator 16 through the force sensation presentation apparatus 36. This enables manipulation based on both visual and tactile sensations and allows the user to manipulate the sample 40 while grasping what kind of force is applied to the sample 40 according to the relative positions of the sample 40 and the manipulator 16. This can consequently help master the skill to control the force applied to the cell properly.

According to the present disclosure, the force sensation determined by the force applied between the sample 40 and the manipulator 16 can be presented by generating force information indicating the magnitude of force sensation to be presented to the user, based on the image capturing the sample 40 manipulated by using the manipulator 16. Since it is difficult to detect the force applied to the sample 40 by the manipulator 16 in real time by using a sensor, etc., generation of force information based on the image makes it possible to present the force sensation in real time and improve the operability.

Some aspects of the present disclosure will be described.

The first aspect of the present disclosure relates to a manipulation system including: a manipulator for manipulating a sample; a manipulator drive mechanism for moving the manipulator; an imaging apparatus for imaging the sample through an objective lens; a control apparatus that identifies, based on an image captured by the imaging apparatus, an amount of change in at least one of a position or a shape of the sample and estimates a force applied between the sample and the manipulator based on the amount of change identified; and a force sensation presentation apparatus configured to receive an input operation from the user for designating a position of the manipulator and to present a force sensation corresponding to the force estimated by the control apparatus to the user.

The second aspect of the present disclosure relates to the manipulation system according to the first aspect, wherein the control apparatus identifies, based on the image, an amount of deformation of the sample in a direction of protrusion of a leading end of the manipulator and estimates the force based on the amount of deformation.

The third aspect of the present disclosure relates to the manipulation system according to the second aspect, wherein the control apparatus identifies the amount of deformation based on a position of the leading end of the manipulator, a central position of the sample, and a position of an end of the sample in the direction of protrusion.

The fourth aspect of the present disclosure relates to the manipulation system according to any one the first through third aspects, wherein the control apparatus identifies an amount of displacement of the sample based on the image and generates the force information based on the amount of displacement.

The fifth aspect of the present disclosure relates to the manipulation system according to any one the first through fourth aspects, wherein the control apparatus transmits force information indicating a force derived from amplifying a magnitude of the force thus estimated by 100 times or more to the force sensation presentation apparatus, and the force sensation presentation apparatus presents the force thus amplified to the user.

The sixth aspect of the present disclosure relates to a manipulation method including: acquiring position information based on a user's input operation for designating a position of a manipulator from a force sensation presentation apparatus; controlling, based on the position information acquired, an operation of a manipulator drive mechanism for moving the manipulator; imagining a sample manipulated by using the manipulator through an objective lens; identifying, based on an image captured, an amount of change in at least one of a position or a shape of the sample; estimating a force applied between the sample and the manipulator based on the amount of change identified; and controlling an operation of the force sensation presentation apparatus so that a force sensation according to the force thus estimated is presented to the user.

The seventh aspect of the present disclosure relates to a program including computer-implemented modules including: a module that acquires position information based on a user's input operation for designating a position of the manipulator from a force sensation presentation apparatus; a module that controls, based on the position information acquired, an operation of a manipulator drive mechanism for moving the manipulator; a module that acquires an image of a sample manipulated by using the manipulator captured through an objective lens; a module that identifies, based on an image captured, an amount of change in at least one of a position or a shape of the sample; a module that estimates a force applied between the sample and the manipulator based on the amount of change; and a module that controls an operation of the force sensation presentation apparatus so that a force sensation corresponding to the force thus estimated is presented to the user.

(Second embodiment)

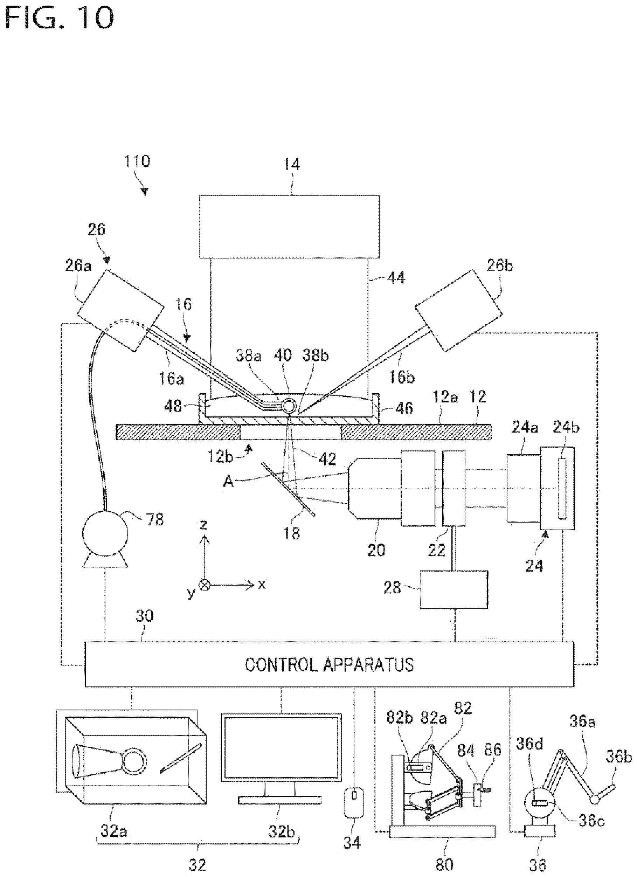

FIG. 10 is a diagram schematically showing a configuration of a manipulation system 110 according to the second embodiment. The second embodiment differs from the first embodiment in that a second force sensation presentation apparatus 80 for manipulating the holding pipette 16a is used apart from the force sensation presentation apparatus 36 (also referred to as the first force sensation presentation apparatus 36) for manipulating the injection pipette 16b. The following description of the second embodiment highlights the difference from the first embodiment. A description of common features is omitted as appropriate.

The manipulation system 110 includes a stage 12, an illumination apparatus 14, a manipulator 16, a redirecting mirror 18, an objective lens 20, a variable focus lens 22, an imaging apparatus 24, a manipulator drive mechanism 26, a lens drive mechanism 28, a control apparatus 30, a display apparatus 32, an input apparatus 34, a first force sensation presentation apparatus 36, a pump 78, and a second force sensation presentation apparatus 80.

The first force sensation presentation apparatus 36 is configured in the same manner as in the first embodiment described above. The first force sensation presentation apparatus 36 is an input operation apparatus for manipulating the injection pipette 16b. The multi-joint arm 36a of the first force sensation presentation apparatus 36 is a first link mechanism for supporting the leading end 36b of the multi-joint arm 36a and is, for example, a serial link mechanism. The leading end 36b of the multi-joint arm 36a is a first holding member held by the user. Therefore, the first force sensation presentation apparatus 36 includes a first holding member held by the user and a first link mechanism that supports the first holding member so that the position of the first holding member is variable according to the user's operation.

The first force sensation presentation apparatus 36 further includes an actuator 36c for driving the first link mechanism (multi-joint arm 36a) to apply a reaction force to the first holding member (leading end 36b). The first force sensation presentation apparatus 36 further includes a position sensor 36d for detecting the orientation of the multi-joint arm 36a and detecting the position of the first holding member (leading end 36b). The first force sensation presentation apparatus 36 is manipulated with, for example, the user's right hand.

The pump 78 is connected to the interior of the holding pipette 16a and generates a suction force or a discharge force at the leading end 38a of the holding pipette 16a. The pump 78 is, for example, an electric microinjector. The pump 78 generates a suction force at the leading end 38a of the holding pipette 16a by producing a negative pressure in the interior of the holding pipette 16a. The pump 78 generates a discharge force at the leading end 38a of the holding pipette 16a by producing a positive pressure in the holding pipette 16a. The pump 78 variably controls the suction force and the discharge force based on a command transmitted from the control apparatus 30.

The second force sensation presentation apparatus 80 is an input operation apparatus for manipulating the manipulator 16 and is an input operation apparatus for manipulating the holding pipette 16a. The second force sensation presentation apparatus 80 is configured to receive an input operation for designating the three-dimensional position of the holding pipette 16a and an input operation for designating the suction force and the discharge force at the end of the holding pipette 16a. The second force sensation presentation apparatus 80 includes a second link mechanism 82, a second holding member 84, and a rotating member 86. The second force sensation presentation apparatus 80 is manipulated with, for example, the user's left hand.

The second link mechanism 82 supports the second holding member 84 so that the position of the second holding member 84 is variable according to the user's operation. The second link mechanism 82 is configured so that the second holding member 84 attached to the leading end of the second link mechanism 82 can be moved along three axes of XYZ. The second link mechanism 82 is, for example, a parallel link mechanism in which three arms are connected in parallel. The second link mechanism 82 is provided with, for example, an actuator 82a for driving each of the three arms to apply a reaction force to the second holding member 84 and a position sensor 82b for detecting the orientation of each of the three arms.

The second holding member 84 is a member held by the user. The second holding member 84 is held with, for example, the user's thumb and middle finger during use. The user performs an input operation to designate the three-dimensional position of the holding pipette 16a by holding the second holding member 84 to move the second holding member 84. The three-dimensional position of the second holding member 84 is detected by, for example, a position sensor 82b provided in the second link mechanism 82.

The rotating member 86 is a member for receiving an input operation from a gripping action of the user. The rotating member 86 is provided in the vicinity of the second holding member 84 and is configured to be rotatable with respect to the second holding member 84. By rotating the rotating member 86, the user performs an input operation to designate a suction force and a discharge force at the leading end 38a of the holding pipette 16a.

For example, Omega 7, manufactured by Force Dimension, can be used as the second force sensation presentation apparatus 80. For example, an active gripper and a force sensation device disclosed in WO 2008/003416 can be used.

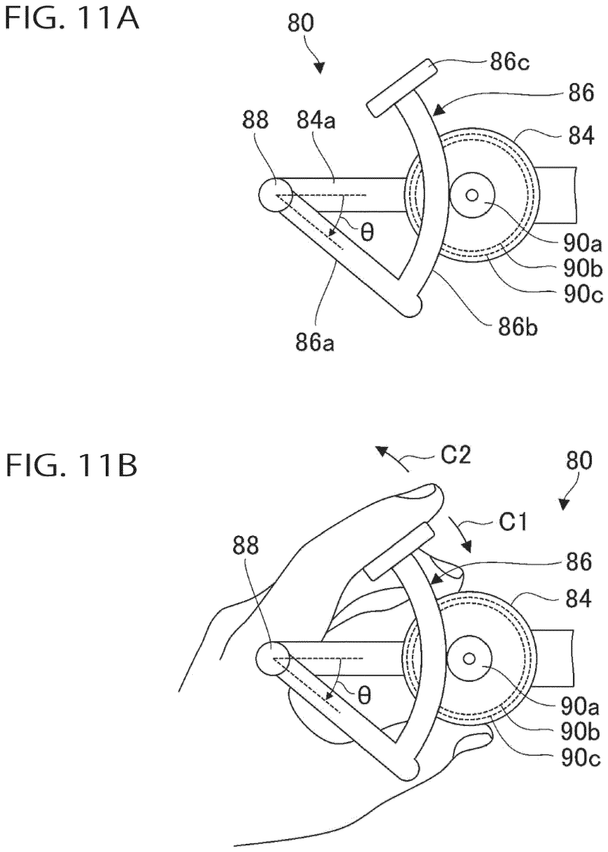

FIGS. 11A-11B are diagrams schematically showing a configuration of the second force sensation presentation apparatus 80. FIG. 11A schematically shows a configuration of the second holding member 84 and the rotating member 86, and FIG. 11B schematically shows an input operation from a gripping action of the user gripping the second holding member 84.

The second holding member 84 has a shape that is easily held with the user's fingers and has, for example, a cylindrical shape. A connection bar 84a extending radially from the second holding member 84 is attached to the second holding member 84. At the leading end of the connection bar 84a is provided a rotating shaft 88 that rotatably supports the rotating member 86.

The rotating member 86 includes a straight member 86a, an arc member 86b, and a pad 86c. The straight member 86a is connected to the rotating shaft 88 and is rotatable around the rotating shaft 88. The arc member 86b extends from the end of the straight member 86a toward the pad 86c along an arc centered around the rotating shaft 88. The pad 86c is provided at the end of the arc member 86b and comes into contact with the user's index finger during use.

The second holding member 84 includes a pulley 90a for driving the rotating member 86, an actuator 90b for driving the pulley 90a, and an angle sensor 90c for detecting the rotation angle of the pulley 90. The pulley 90a is configured to mesh with the outer circumference of the arc member 86b. When the arc member 86b moves in the circumferential direction due to the rotation of the rotating member 86, the pulley 90a rotates according to the amount of movement in the circumferential direction. The actuator 90b applies a reaction force to the rotating member 86 by driving the pulley 90a to present a force sensation to the user manipulating the rotating member 86. The angle sensor 90c detects the rotation angle $\theta$ of the rotating member 86 with respect to the second holding member 84 by detecting the rotation angle of the pulley 90a.

When the user performs a gripping action as if to grip an object with a finger, i.e., a gripping action of bringing the thumb and the index finger to approach and face each other, as indicated by the arrow C1 in FIG. 11B, the rotating member 86 rotates clockwise with respect to the second holding member 84, and the rotation angle $\theta$ increases. When, as shown by the arrow C2 in FIG. 11C, the user performs a gripping action as if to release the gripped object, i.e., a gripping action that opens the space between the thumb and the index finger, on the other hand, the rotating member 86 rotates counterclockwise with respect to the second holding member 84, and the rotation angle $\theta$ decreases.

The control apparatus 30 acquires position information indicating the three-dimensional position of the second holding member 84 from the second force sensation presentation apparatus 80. The control apparatus 30 controls the operation of the first drive mechanism 26a that moves the holding pipette 16a, based on the position information acquired from the second force sensation presentation apparatus 80.

The control apparatus 30 acquires angle information indicating the rotation angle $\theta$ of the rotating member 86 from the second force sensation presentation apparatus 80. The control apparatus 30 controls the operation of the pump 78 that variably controls the suction force and the discharge force at the leading end 38a of the holding pipette 16a, based on the angle information acquired from the second force sensation presentation apparatus 80. The angle information indicating the rotation angle $\theta$ of the rotating member 86 is an example of manipulation information indicating a magnitude of manipulation that designates the suction force and the discharge force of the pump 78.

When the rotation angle $\theta$ of the rotating member 86 is a predetermined initial value $\theta_0$ (i.e., $\theta=\theta_0$), the control apparatus 30 ensures that neither a suction force nor a discharge force is generated at the leading end 38a of the manipulator 16. That is, the operation of the pump 78 is stopped when the rotation angle $\theta$ of the rotating member 86 is the predetermined initial value $\theta_0$.

When the rotation angle $\theta$ of the rotating member 86 is greater than the predetermined initial value $\theta 0$ (i.e., $\theta>\theta_0$), the control apparatus 30 actuates the pump 78 so that a suction force is generated at the leading end 38a of the manipulator 16. The control apparatus 30 may actuate the pump 78 so that the suction force varies according to the rotation angle $\theta$ of the rotating member 86 and may ensure that the suction force increases as the rotation angle $\theta$ of the rotating member 86 increases. For example, the suction force may be proportional to the magnitude of a difference $\Delta\theta$ ($=\theta-\theta_0$) between the rotation angle $\theta$ and the initial value $\theta_0$ of the rotating member 86.

When the rotation angle $\theta$ of the rotating member 86 is smaller than the predetermined initial value $\theta_0$ (i.e., $\theta<\theta_0$), the control apparatus 30 actuates the pump 78 so that a discharge force is generated at the leading end 38a of the manipulator 16. The control apparatus 30 may actuate the pump 78 so that the discharge force varies according to the rotation angle $\theta$ of the rotating member 86, and the discharge force may increase as the rotation angle $\theta$ of the rotating member 86 decreases. For example, the discharge force may be proportional to the magnitude of the difference $\Delta\theta(=\theta-\theta_0)$ between the rotation angle $\theta$ and the initial value $\theta_0$ of the rotating member 86.

The control apparatus 30 generates force information indicating the magnitude and direction of the force sensation presented to the user through the second force sensation presentation apparatus 80. The control apparatus 30 generates force information so that the force sensation corresponding to the suction force or the discharge force at the leading end 38a of the holding pipette 16a is fed back.

The control apparatus 30 generates force information that sets the magnitude of force to 0, when the rotation angle of the rotating member 86 is the predetermined initial value $\theta_0$ (i.e., $\theta=\theta_0$), and neither a suction force nor a discharge force is generated. Therefore, it is ensured that a force sensation is not presented to the user when neither a suction force nor a discharge force is generated. This makes it possible to covey to the user through a force sensation whether the rotation angle $\theta$ of the rotating member 86 is the predetermined initial value $\theta_0$.

The control apparatus 30 generates force information indicating the magnitude of force corresponding to the suction force when the rotation angle $\theta$ of the rotating member 86 is larger than the predetermined initial value $\theta_0$ (i.e., $\theta>\theta_0$), and a suction force is generated. The control apparatus 30 generates force information in the direction opposite to the rotation direction of the rotating member 86. Thus, when the rotating member 86 is rotated in the direction of the arrow C1 in FIG. 11B, a force sensation in the opposite direction indicated by the arrow C2 is generated so that a force sensation felt as a reaction force can be presented to the user. It can also convey to the user through a force sensation that the suction force is increasing according to the magnitude of manipulation of the rotating member 86, by increasing the reaction force as the magnitude of the difference $\Delta\theta(=\theta-\theta_0)$ between the rotation angle $\theta$ and the initial value $\theta_0$ of the rotating member 86 increases, i.e., as the suction force increases. The control apparatus 30 may generate force information for a magnitude proportional to the difference $\Delta\theta(=\theta-\theta_0)$ between the rotation angle $\theta$ and the initial value $\theta_0$ of the rotating member 86. The control apparatus 30 may generate force information for a magnitude proportional to the suction force of the pump 78.

The control apparatus 30 generates force information indicating the magnitude of force corresponding to the discharge force when the rotation angle $\theta$ of the rotating member 86 is smaller than the predetermined initial value $\theta_0$ (i.e., $\theta<\theta_0$) and a discharge force is generated. The control apparatus 30 generates force information in the direction opposite to the rotation direction of the rotating member 86. Thus, when the rotating member 86 is rotated in the direction of the arrow C2 in FIG. 11B, a force sensation in the opposite direction indicated by the arrow C1 is generated so that a force sensation felt as a reaction force can be presented to the user. It can also convey to the user through a force sensation that the discharge force is increasing according to the magnitude of manipulation of the rotating member 86, by increasing the reaction force as the magnitude of the difference $\Delta\theta(=\theta-\theta_0)$ between the rotation angle $\theta$ and the initial value $\theta_0$ of the rotating member 86 increases, i.e., as the suction force increases. The control apparatus 30 may generate force information for a magnitude proportional to the difference $\Delta\theta(=\theta-\theta_0)$ between the rotation angle $\theta$ and the initial value $\theta_0$ of the rotating member 86. The control apparatus 30 may generate force information for a magnitude proportional to the discharge force of the pump 78.

The control apparatus 30 may generate force information indicating different magnitudes of reaction force depending on whether the sample 40 is in contact with the leading end 38a of the holding pipette 16a. When the suction force or the discharge force is assumed to be constant, for example, the reaction force generated when the sample 40 is in contact with the leading end 38a of the holding pipette 16a may be configured to be greater than the reaction force generated when the sample 40 is not in contact with the leading end 38a of the holding pipette 16a. In this case, the reaction force increases at the moment when the sample 40 comes into contact with the leading end 38a of the holding pipette 16a so that it is possible to convey to the user through a force sensation that the sample 40 has come into contact. Further, the reaction force decreases at the moment when the sample 40 is detached from the leading end 38a of the holding pipette 16a so that it is possible to convey to the user through a force sensation that the sample 40 has been detached.

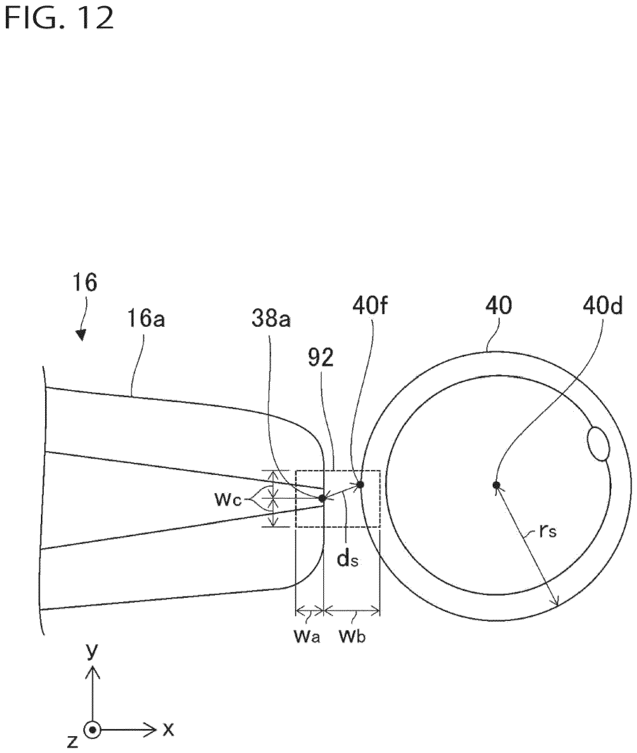

FIG. 12 is a diagram schematically showing a method for determining a contact between the sample 40 and the manipulator 16. The control apparatus 30 determines whether the sample 40 is in contact with the leading end 38a of the holding pipette 16a based on the captured image of the sample 40. The control apparatus 30 makes a contact determination based on the position of the sample 40 with respect to the position of the leading end 38a of the holding pipette 16a. The position of the sample 40 can be identified based on the captured image of the sample 40. The position of the leading end 38a of the holding pipette 16a can be identified by the method using coordinate conversion described in the three-dimensional position identification method according to the first embodiment.

The control apparatus 30 makes a contact determination according to whether an end 40f of the sample 40 is included within the range of a neighboring area 92 of the leading end 38a of the holding pipette 16a. The neighboring area 92 indicated by a dashed line in FIG. 12 is, for example, set in a range having a width wb in the direction of protrusion (+x direction) of the holding pipette 16a, a width wa in a direction (-x direction) opposite to the direction of protrusion, and a width wc in a direction ($\pm$y direction) perpendicular to the direction of protrusion with reference to the leading end 38a of the holding pipette 16a. The size of the neighboring area 92 can be set to be about the same as, for example, the size of the leading end 38a of the holding pipette 16a and can be set to be equal to or less than the size (radius rs) of the sample 40. By way of example, wa=10 $\mu$, wb=20 $\mu$m, and wc=10 um, and the size of the neighboring area 92 is 30 $\mu$m$\times$20 um, given that the radius rs of the sample 40 is about 50 $\mu$m.

The control apparatus 30 identifies the position coordinates (Cx, Cy) of the center 40d of the sample 40 and the radius rs of the sample 40 based on the captured image. The control apparatus 30 identifies the position coordinates of the leading end 38a of the holding pipette 16a in the second coordinate system by referring to the operation of the manipulator drive mechanism 26 and identifies the position coordinates (Mx, My) of the leading end 38a by coordinate conversion from the second coordinate system to the first coordinate system. The control apparatus 30 calculates the position coordinates (Cx-rs, Cy) of the end 40f of the sample 40 from the position coordinates (Cx, Cy) of the center 40d of the sample 40 and the radius rs of the sample 40. The control apparatus 30 calculates the range of the neighboring area 92 from the position coordinates (Mx, My) of the leading end 38a of the holding pipette 16a. The control apparatus 30 determines that the sample 40 is in contact with the leading end 38a of the holding pipette 16a when the end 40f of the sample 40 is within the range of the neighboring area 92. Specifically, the control apparatus 30 determines that the sample 40 is in contact with the leading end 38a of the holding pipette 16a when $Mx-wa \leq Cx-rs \leq Mx+wb$ and $My-wc \leq Cy \leq My+wc$. The control apparatus 30 determines that the sample 40 is not in contact with the leading end 38a of the holding pipette 16a when the end 40f of the sample 40 is outside the range of the neighboring area 92.

The control apparatus 30 defines a value $k_1\Delta\theta$ derived from multiplying the difference $\Delta\theta(=\theta-\theta_0)$ between the rotation angle $\theta$ and the initial value $\theta_0$ of the rotating member 86 by the first coefficient $k_1$ to be magnitude of the reaction force, when the control apparatus 30 determines that the sample 40 is not in contact with the leading end 38a of the holding pipette 16a. The control apparatus 30 defines a value $k_2\Delta\theta$ derived from multiplying the difference $\Delta\theta(=\theta-\theta_0)$ between the rotation angle $\theta$ and the initial value $\theta_0$ of the rotating member 86 by the second coefficient $k_2$ to be the magnitude of the reaction force, when the control apparatus 30 determines that the sample 40 is not in contact with the leading end 38a of the holding pipette 16a. The second coefficient $k_2$ is larger than the first coefficient $k_1$ (i.e., $k_2>k_1$). The ratio $k_2/k_1$ between the first coefficient $k_1$ and the second coefficient $k_2$ is, for example, 1.1 or more and 5 or less and is, preferably, 1.5 or more and 3 or less. By way of example, the ratio is 2. The first coefficient $k_1$ and the second coefficient $k_2$ can be set so that the maximum value of the reaction force applied to the rotating member 86 is 0.5 N or more and 5 N or less and is, preferably, 1 N or more and 2 or less.

It should be noted that, instead of switching the magnitude of the reaction force according to whether the sample 40 is in contact with the leading end 38a of the holding pipette 16a, the magnitude of the reaction force may be changed continuously according to a distance ds from the leading end 38a of the holding pipette 16a to the end 40f of the sample 40. For example, the magnitude of the reaction force may be decreased as the distance ds from the leading end 38a of the holding pipette 16a to the sample 40 increases, given that the suction force or the discharge force is constant. For example, the magnitude of the reaction force may be defined to be $f(ds)\Delta\theta$, using a sigmoid function $f(ds)$ that equals the second coefficient $k_2$ when the distance ds is 0 and equals the first coefficient $k_1$ when the distance ds is infinite.

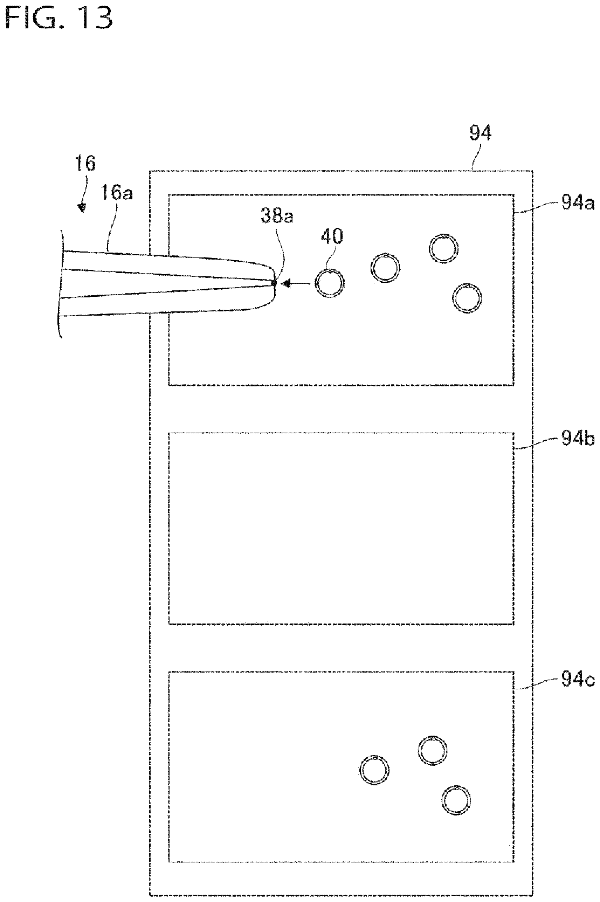

A description will now be given, with reference to FIGS. 13-15, of the flow of manipulation of the sample 40 by the holding pipette 16a. FIGS. 13-15 show work areas 94 for manipulating the sample 40. The work area 94 is roughly divided into three areas 94a, 94b, and 94c. The first area 94a is an area where an untreated sample 40 not subjected to cell manipulation yet is arranged. The second area 94b is an area where cell manipulation on the sample 40 is performed by using the injection pipette 16b. The third area 94c is an area in which the treated sample 40 subjected to cell manipulation is arranged.

First, as shown in FIG. 13, the holding pipette 16a is moved to the first area 94a, and the leading end 38a of the holding pipette 16a is brought into close proximity to the sample 40 located in the first area 94a. The user can move the leading end 38a of the holding pipette 16a to the first area 94a by holding and moving the second holding member 84 of the second force sensation presentation apparatus 80.

A suction force is then generated at the leading end 38a of the holding pipette 16a in the vicinity of the sample 40 to suck and hold the sample 40 at the leading end 38a of the holding pipette 16a. The user generates a suction force at the leading end 38a of the holding pipette 16a by performing a gripping action so that the rotation angle $\theta$ of the rotating member 86 increases. The user can grasp the magnitude of the suction force according to the reaction force applied to the rotating member 86. Further, when the sample 40 comes into contact with the leading end 38a of the holding pipette 16a and is held by the suction force, the reaction force applied to the rotating member 86 increases. Therefore, the user can check whether the sample 40 is being held at the leading end 38a of the holding pipette 16a according to a change in the reaction force applied to the rotating member 86. The user can obtain a force sensation as if the user is grasping and holding the sample 40 directly with the hand according to the reaction force applied to the rotating member 86.

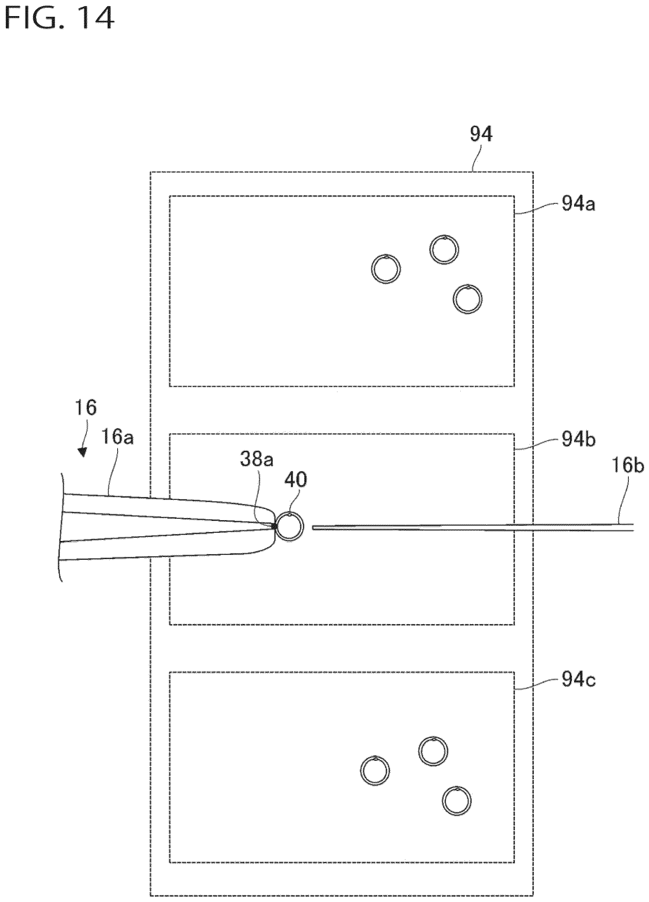

Subsequently, as shown in FIG. 14, the user moves the holding pipette 16a from the first area 94a to the second area 94b while holding the sample 40. The user can move the sample 40 held at the leading end 38a of the holding pipette 16a to the second area 94b by moving the second holding member 84 while maintaining a gripping action of gripping the rotating member 86. The holding pipette 16a may not retain the sample 40 while moving, and the sample 40 may come off from the leading end 38a. In this case, it is determined that the sample 40 is not in contact and the reaction force applied to the rotating member 86 is reduced so that the user can check that the sample 40 has come off from the leading end 38a of the holding pipette 16a by sensing a decrease in the reaction force applied to the rotating member 86.

Next, in the second area 94b, cell manipulation is performed by using the injection pipette 16b on the sample 40 held at the leading end 38a of the holding pipette 16a. For example, the user can manipulate the holding pipette 16a with the left hand through the second force sensation presentation apparatus 80 while manipulating the injection pipette 16b with the right hand through the first force sensation presentation apparatus 36.

For example, the user performs cell manipulation as shown in FIGS. 8B and 8C using the injection pipette 16b. In the rotation operation of FIG. 8B, the user can perform an operation of moving the injection pipette 16b with the right hand while adjusting the suction force by changing the rotation angle $\theta$ of the rotating member 86 with the left hand. In the piecing operation of FIG. 8C, the user can not only move the injection pipette 16b but move the holding pipette 16a to adjust the position of the sample 40 or adjust the suction force on the sample 40 at the same time. In this process, the user can check through a force sensation whether the sample 40 is successfully held by the holding pipette 16a according to a change in the reaction force applied to the rotating member 86.

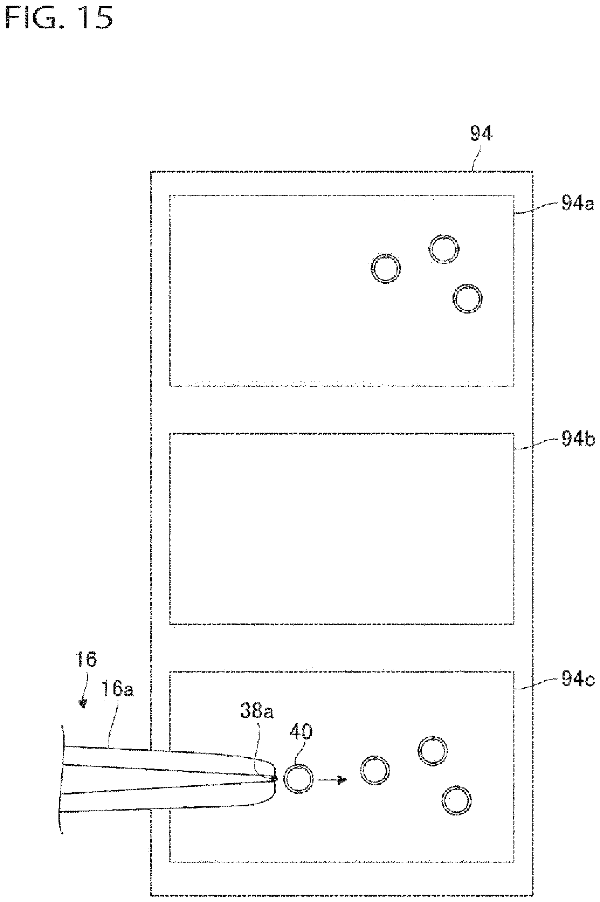

Subsequently, as shown in FIG. 15, the user moves the holding pipette 16a from the second area 94b to the third area 94c while holding the sample 40 for which cell manipulation has been completed. The user can move the sample 40 held at the leading end 38a of the holding pipette 16a to the third area 94c by moving the second holding member 84 while maintaining a gripping action of gripping the rotating member 86.

Subsequently, a discharge force is generated at the leading end 38a of the holding pipette 16a in the third area 94c. The user can generate a discharge force at the leading end 38a of the holding pipette 16a by performing a gripping action of opening the index finger to reduce the rotation angle $\theta$ of the rotating member 86. The user can check the magnitude of the discharge force according to the reaction force applied to the rotating member 86. The sample 40 comes off from the leading end 38a of the holding pipette 16a due to the discharge force and is released. When the sample 40 comes off from the leading end 38a of the holding pipette 16a and is not in contact any more, the reaction force applied to the rotating member 86 becomes smaller so that the user can check that the sample 40 has come off from the leading end 38a of the holding pipette 16a by sensing a decrease in the reaction force applied to the rotating member 86.

When cell manipulation on the next sample 40 is necessary, the holding pipette 16a may be moved from the third area 94c to the first area 94a. When cell manipulation is performed on a plurality of samples 40 in sequence, the manipulations of FIGS. 13-15 are performed repeatedly.

According to the present disclosure, the manipulation to move the holding pipette 16a and the manipulation for suction and discharge of the sample 40 by the holding pipette 16a can be realized with a single input operation apparatus by using the second force sensation presentation apparatus 80. When, for example, an operation to move the holding pipette 16a by using a joystick is performed, as in the first embodiment, it is necessary to use a manipulation means other than the joystick for suction and discharge, which requires a step of switching the manipulation means. If the sample 40 comes off while the holding pipette 16a retaining the sample 40 is moved, it requires switching of the manipulation means to hold the sample 40 again, causing the operation to become complicated. According to this embodiment, on the other hand, only a single input operation apparatus need be used so that switching of the manipulation means is not necessary, and the operability can be improved.

According to the present disclosure, the suction force and the discharge force at the leading end 38a of the holding pipette 16a can be presented to the user as a force sensation by using the second force sensation presentation apparatus 80. The user can adjust the magnitude of the suction force or the discharge force while feeling a force sensation determined by the magnitude of the suction force or the discharge force so that the operability of suction and discharge can be improved.

According to the present disclosure, whether the sample 40 is held at the leading end 38a of the holding pipette 16a can be presented to the user as a force sensation by determining whether the sample 40 is contact with the leading end 38a of the holding pipette 16a. As a result, it is easier to check whether the sample 40 is held by the holding pipette 16a than in the case of visual inspection only, and the operability experienced by the user holding the sample 40 or releasing the sample 40 can be improved.

According to the present disclosure, a force sensation as if the user is grasping the sample 40 directly to hold the sample 40 can be presented by generating a suction force by a gripping action in the direction of gripping the rotating member 86 and applying a reaction force corresponding to the suction force to the rotating member 86. Further, a force sensation simulating the moment when the sample 40 is directly touched by the hand can be presented by increasing the reaction force when the sample 40 comes into contact with the leading end 38a of the holding pipette 16a and is held accordingly. The reaction force decreases when the sample 40 comes off from the leading end 38a of the holding pipette 16a so that a force sensation simulating the moment when the sample 40 grasped by the hand is released can be presented. As a result, it is possible to present a force sensation that is not so uncomfortable for the user, and the operability of suction and discharge can be improved more effectively.

In order to check the effectiveness of the manipulation system 110 according to the present disclosure, a demonstration experiment to measure the work time required for a series of operations shown in FIGS. 13-15 was conducted. In the demonstration experiment, microbeads were used as the sample 40, and the time required for an operation of moving three microbeads from the first area 94a to the third area 93 was measured. In a comparative example, the joystick shown in the first embodiment was used as the input operation apparatus for manipulating the holding pipette 16a. In Example 1, the second force sensation presentation apparatus 80 was used as the input operation apparatus, and a force sensation through the rotating member 86 was not presented. In Example 2, the second force sensation presentation apparatus 80 was used as the input operation apparatus, and a force sensation through the rotating member 86 was presented.

In each of the comparative example, Example 1, and Example 2, the average value of time required by 6 adults who had no experience of cell manipulation was determined. In the comparative example, it was 116 seconds. In Example 1, it was 51 seconds. In Example 2, it was 42 seconds. The p-value of Welch's T-test was less than 0.01 between the comparative example and Example 1 and between Example 1 and Example 2. It was found from this that the operability can be significantly improved by using the second force sensation presentation apparatus 80 as the input operation apparatus instead of a joystick. It was also found that the operability can be significantly improved by applying a reaction force to the rotating member 86 to present a force sensation when the second force sensation presentation apparatus 80 is used as the input operation apparatus.

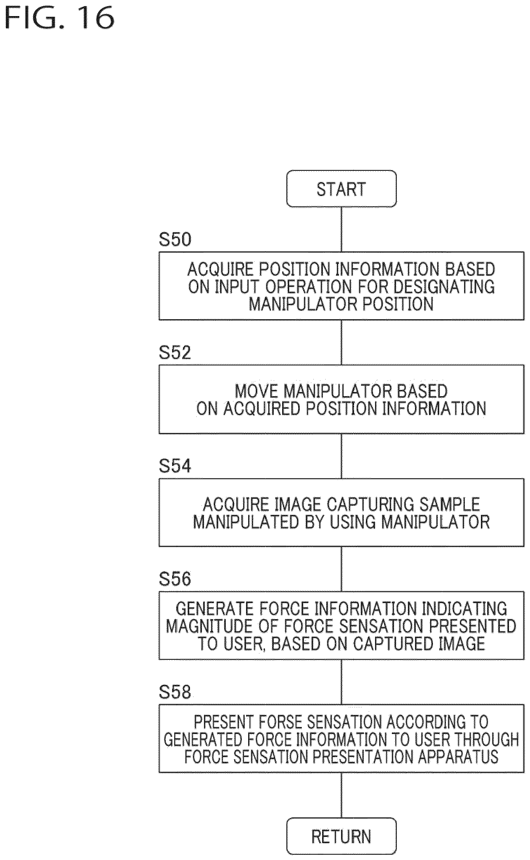

FIG. 16 is a flowchart showing an example of the manipulation method according to the present disclosure. The control apparatus 30 acquires position information based on the user's input operation for designating the position of the manipulator 16 from at least one of the first force sensation presentation apparatus 36 or the second force sensation presentation apparatus 80 (S50). The control apparatus 30 controls the operation of the manipulator drive mechanism 26 based on the acquired position information to move the manipulator 16 (S52). The imaging apparatus 24 images the sample 40 manipulated by using the manipulator 16 through the objective lens 20 (S54). The control apparatus 30 generates force information indicating the magnitude of force sensation presented to the user based on the image captured by the imaging apparatus 24 (S56). The control apparatus 30 controls the operation of at least one of the first force sensation presentation apparatus 36 and the second force sensation presentation apparatus 80 so that a force sensation according to the generated force information is presented to the user (S58).

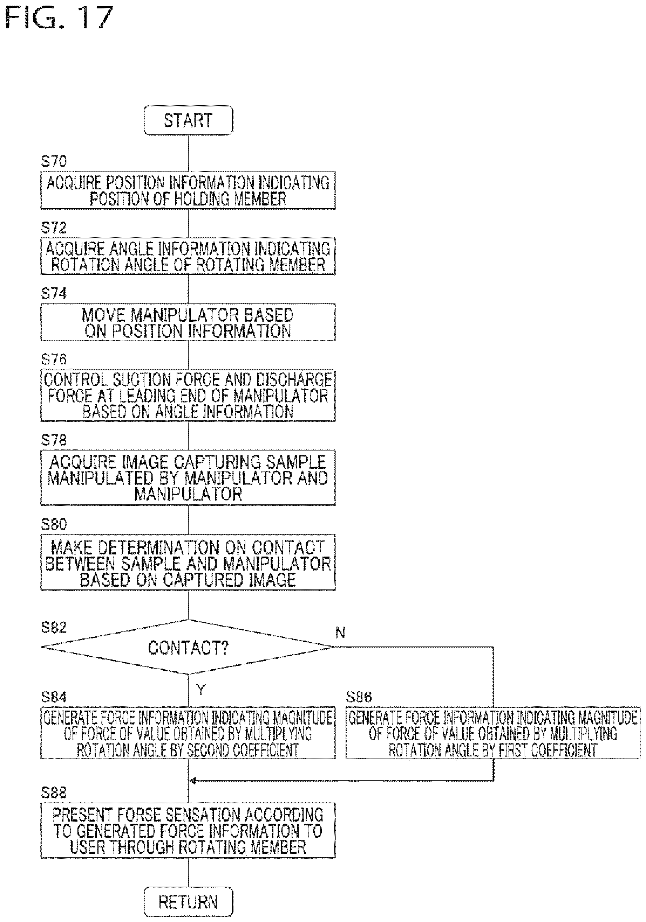

FIG. 17 is a flowchart showing an example of the manipulation method according to the present disclosure. The control apparatus 30 acquires position information indicating the position of the second holding member 84 from the second force sensation presentation apparatus 80 (S70). The control apparatus 30 acquires angle information indicating the rotation angle of the rotating member 86 from the second force sensation presentation apparatus 80 (S72). The control apparatus 30 controls the operation of the manipulator drive mechanism 26 based on the acquired position information to move the manipulator 16 (S74). The control apparatus 30 controls the operation of the pump 78 and controls the suction force and the discharge force at the leading end of the manipulator 16 based on the acquired angle information (S76). The imaging apparatus 24 images the sample 40 manipulated by using the manipulator 16 through the objective lens 20 (S78). The control apparatus 30 determines whether the sample 40 and the manipulator 16 are in contact based on the image captured by the imaging apparatus 24 (S80).

When the sample 40 and the manipulator 16 are in contact (Y in S82), the control apparatus 30 generates force information indicating the magnitude of force of a value obtained by multiplying the rotation angle $(\theta-\theta_0)$ by the second coefficient $k_2$ (S84). When the sample 40 and the manipulator 16 are not in contact (N in S82), the control apparatus 30 generates force information indicating the magnitude of force of a value obtained by multiplying the rotation angle $(\theta-\theta_0)$ by the first coefficient $k_1$ (S86). The second coefficient $k_2$ may be greater than the first coefficient $k_1$ (i.e., $k_2 > k_1$). The control apparatus 30 controls the operation of the second force sensation presentation apparatus 80 so that a force sensation according to the generated force information is presented to the user through the rotating member 86 (S88).

In the flow of FIG. 17, the processes in S80-S88 for presenting a force sensation to the user may be omitted. Even in the case the user is not presented with a force sensation, the operability of the user can be improved as demonstrated in Example 1 described above.

(Third embodiment)

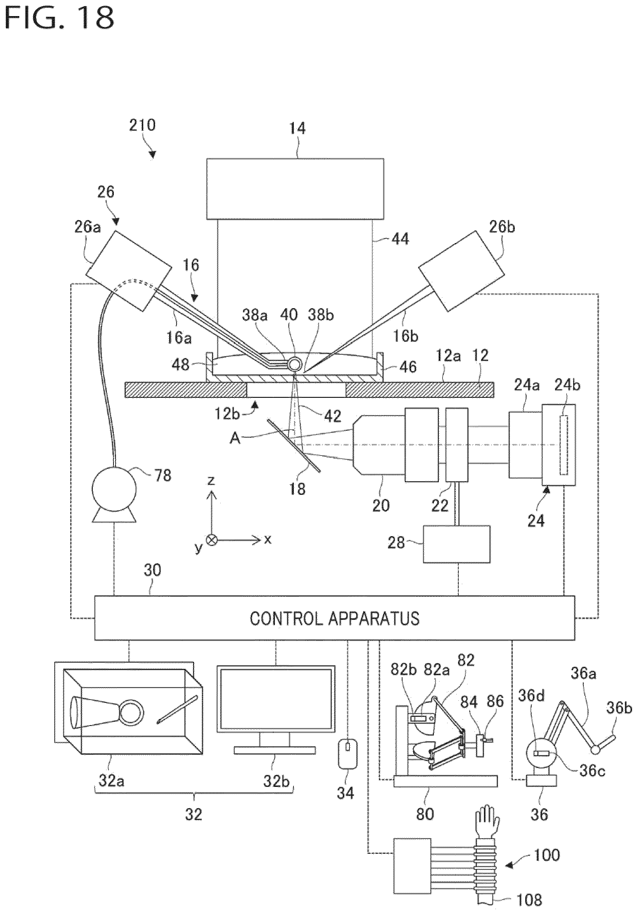

FIG. 18 is a diagram schematically showing a configuration of a manipulation system 210 according to the third embodiment. The third embodiment differs from the second embodiment described above in that a third force sensation presentation apparatus 100 for presenting the user with a force sensation received by the sample 40 due to the manipulation by the manipulator 16 is further used. The following description of the third embodiment highlights the difference from the second embodiment. A description of common features is omitted as appropriate.

The manipulation system 210 includes a stage 12, an illumination apparatus 14, a manipulator 16, a redirecting mirror 18, an objective lens 20, a variable focus lens 22, an imaging apparatus 24, a manipulator drive mechanism 26, a lens drive mechanism 28, a control apparatus 30, a display apparatus 32, an input apparatus 34, a first force sensation presentation apparatus 36, a pump 78, a second force sensation presentation apparatus 80, and a third force sensation presentation apparatus 100.

The third force sensation presentation apparatus 100 is attached to the user's body and is configured to present a planar force sensation to the user's body surface. The third force sensation presentation apparatus 100 is, for example, attached to a user's forearm 108 and configured to apply a force as if to tighten the forearm. The third force sensation presentation apparatus 100 presents a force sensation according to the deformation of the sample 40 manipulated by the manipulator 16. The third force sensation presentation apparatus 100 simulates a force sensation that the sample 40 may be experiencing by the manipulation by the manipulator 16. The user can experience a change in the shape of the sample 40 subject to manipulation as a tactile sensation of a force that tightens the user, by using the third force sensation presentation apparatus 100. This makes it easy to grasp the extensibility of the sample 40.

The extensibility of the sample 40 refers to the degree of deformation of the sample 40 that occurs until the injection pipette 16b penetrates the zona pellucida 40b of the sample 40 to pierce the sample 40 it in the piercing operation on the sample 40 shown in FIG. 8C. The case where the extensibility of the sample 40 is high represents a case where the zona pellucida 40b is relatively flexible and the sample 40 is deformed so as to be concave significantly before being pierced. Conversely, the case where the extensibility of the sample 40 is low represents a case where the sample is pierced without being deformed so much.

It is known that there is a high possibility that denaturation will occur in the sample 40 after cell manipulation if the extensibility of the sample 40 is low. Therefore, it is desirable to be able to check the extensibility of the sample 40 during cell manipulation and to know the soundness of the sample 40. If a plurality of piercing operations are performed on the sample 40 in order to check the extensibility of the sample 40, on the other hand, the sample 40 will be damaged. It is therefore required to ensure that the extensibility of the sample 40 is known in one piercing operation. In this embodiment, the degree of deformation of the sample 40 is presented to the user as a force sensation, thereby supporting the understanding of the extensibility of the sample 40.

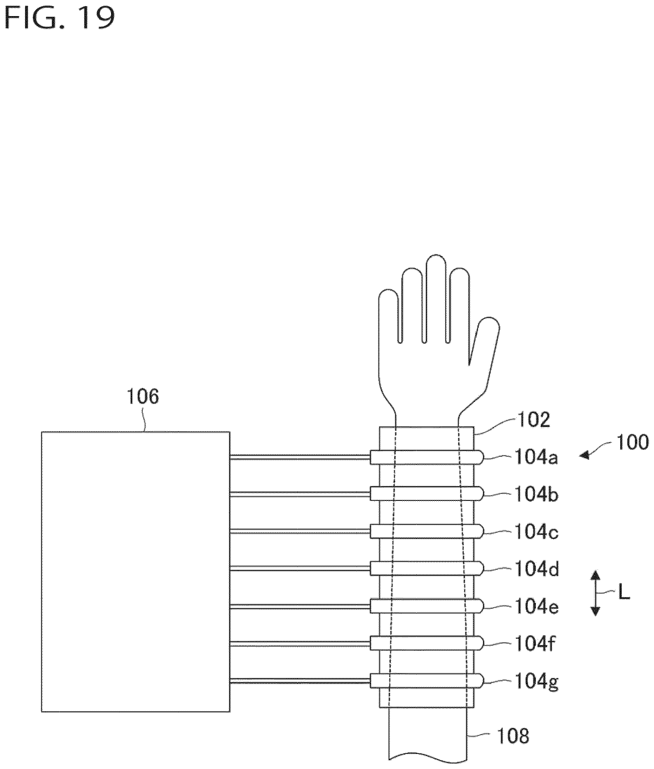

FIG. 19 is a diagram schematically showing a configuration of the third force sensation presentation apparatus 100. The third force sensation presentation apparatus 100 includes an immobilizing brace 102, a plurality of actuators 104a, 104b, 104c, 104d, 104e, 104f, 104g, and a drive control apparatus 106.

The immobilizing brace 102 is a member for fixing the plurality of actuators 104a-104g to the user's body surface. The immobilizing brace 102 attached to the user's forearm 108 is exemplified by an arm cover made of a cylindrical elastic cloth. The immobilizing brace 102 may have a band, etc. for wrapping the immobilizing brace 102 around the user's body surface and fixing it thereto.

The plurality of actuators 104a-104g include, for example, a MckibbenIt type artificial muscle and has an elastic member such as a rubber tube driven by air pressure. The plurality of actuators 104a-104g are attached to the immobilizing brace 102 such that the actuators are wrapped around the outer circumference of the cylindrical immobilizing brace 102. The plurality of actuators 104a-104g apply a force to tighten the immobilizing brace 102 in response to an increased air pressure. The plurality of actuators 104a-104g are arranged at intervals in the axial direction L of the cylindrical immobilizing brace 102 and apply force to a plurality of different locations in the axial direction L. In the illustrated example, seven actuators 104a-104g are arranged at 30 mm intervals in the axial direction L, but the number of actuators and the interval are not particularly limited.

The drive control apparatus 106 drives the plurality of actuators 104a-104g. The drive control apparatus 106 includes, for example, a compressor for generating compressed air, a tank for accumulating compressed air, and a plurality of electropneumatic regulators that control the air pressure supplied to each of the plurality of actuators 104a-104g. The drive control apparatus 106 variably controls the magnitude of force applied by the plurality of actuators 104a-104g to a plurality of locations on the user's body surface by changing the air pressure supplied to each of the plurality of actuators 104a-104g. The drive control apparatus 106 independently and variably controls the magnitude of force applied by each of the plurality of actuators 104a-104g. This makes it possible to present a force sensation in which the magnitude of force could differ in each of the plurality of locations on the surface of the user's body.

Referring back to FIG. 18, the control apparatus 30 generates a plurality of items of force information for controlling the operation of the third force sensation presentation apparatus 100. The control apparatus 30 generates a plurality of items of force information for driving the plurality of actuators 104a-104g. The control apparatus 30 identifies the amount of deformation of the sample 40 at a plurality of positions based on the captured image of the sample 40. The control apparatus 30 generates a plurality of items of force information corresponding to the amount of deformation at each of the plurality of positions based on the amount of deformation of the sample 40 identified at the plurality of positions.

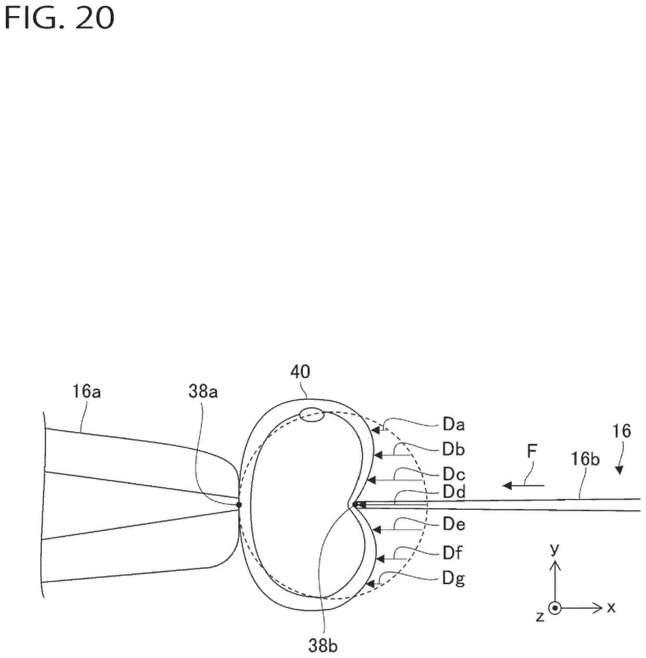

FIG. 20 is a diagram schematically showing the amount of deformation Da-Dg at a plurality of positions of the sample 40 manipulated by the manipulator 16. FIG. 20 shows a piercing operation on the sample 40 and shows the outline of the sample 40 before the piercing operation with a dashed line. The sample 40 is deformed as if it is crushed in the x direction in response to a force F applied in a piecing operation in the x direction by the injection pipette 16b.

The control apparatus 30 identifies, based on the captured image, the amount of deformation Da, Db, Dc, Dd, De, Df, Dg of the sample 40 at a plurality of different positions in the y direction perpendicular to the piercing direction (-x direction). The control apparatus 30 generates a plurality of items of force information according to the identified amount of deformation Da-Dg. The control apparatus 30 generates, for example, a plurality of items of force information indicating the magnitude of force proportional to the identified amount of deformation Da-Dg. In this case, the larger the identified amount of deformation Da-Dg, the greater the magnitude of force indicated by the generated information. By operating the third force sensation presentation apparatus 100 based on such force information, it is possible to present a planar tactile sensation of a force as if the user's forearm 108 is crushed in the same manner as the shape of the sample 40. When the third force sensation presentation apparatus 100 is attached to the user's left arm and the injection pipette 16b is manipulated with the user's right hand, for example, the user can manipulate the sample 40 with the right hand while sensing the degree of deformation of the sample 40 with the left arm.

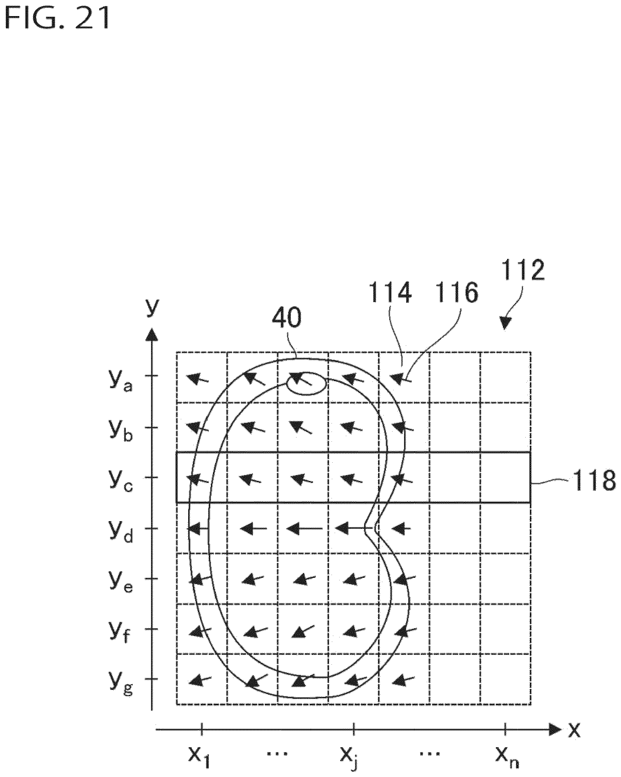

FIG. 21 is a diagram schematically showing a method for calculating the amount of deformation of the sample 40 by using an optical flow. The control apparatus 30 can calculate the amount of deformation Da-Dg of the sample 40 in real time by calculating the optical flow of the captured image. The control apparatus 30 divides a range 112 in the captured image including the sample 40 subject to piercing operation into a plurality of micro areas 114 and calculates the velocity vector 116 in each micro area 114 by using a known optical flow method such as Lucas-Kanade method. The velocity vector 116 indicates the direction and magnitude of a change in image between the image captured at time t-1 and the image captured at time t. The number of divisions in the y direction into the plurality of micro areas 114 is set to 7 according to the number of plurality of actuators 104a-104g. The number n of divisions in the x direction into the plurality of micro areas 114 is not particularly limited but can be set to be equal to the number of divisions as in the y direction. The velocity vector 116 has a component in the x direction and a component in the y direction.

The control apparatus 30 calculates amount of deformation $\Delta D$ of the sample 40 in the x-direction at a particular position in the y direction, by adding up the x component of the velocity vector 116 over a range x1-xn for each position in the y direction. For example, control apparatus 30 calculates the amount of deformation $\Delta Dc$ of the sample 40 in the x-direction at the third position yc from top of FIG. 21 in the y direction, by adding, at the position yc, all the x components of the velocity vector 116 in the range x1-xn bounded by a rectangular frame 118. The control apparatus 30 calculates the amount of deformation $\Delta Di$ (i=a-g) in the x direction at a plurality of y positions yi (i=a-g) by adding the x components of the velocity vector 116 at each of the plurality of y positions yi (i=a-g). The amount of deformation $\Delta Di$ represents the amount of micro deformation of the sample 40 between time t-1 and time t.

The control apparatus 30 calculates the amount of deformation $\Delta Di$ in the x direction at a plurality of y positions yi each time the captured image is acquired. The control apparatus 30 calculates the amount deformation Di (i=a-g) of the sample 40 from the start time t=0 to the current time t by time integration of the amount of deformation ΔDi of the sample 40 in the x direction calculated from the start time t=0 of the piercing operation on the sample 40 until the current time t. In this way, the control apparatus 30 can calculate the amount of deformation Di (i=a-g) of the sample 40 in the x direction by using the optical flow of the captured image.

The control apparatus 30 generates a plurality of force information according to the amount of deformation Di, based on the amount of deformation Di of the sample 40 in the x direction at a plurality of y positions yi. The control apparatus 30 calculates, for example, an air pressure Pi (i=a-g) for driving the plurality of actuators 104a-104g as the plurality of items of force information. The air pressure Pi can be proportional to the amount of deformation Di. For example, $Pi=\beta Di$, where $\beta$ is a proportionality constant. The control apparatus 30 may ensure that the air pressure Pi=0 when the amount of deformation Di is negative, i.e., when the size of the sample 40 is calculated to be larger than in the initial state. The control apparatus 30 may make the air pressure Pi equal to a predetermined upper limit value P0 when the amount of deformation Di exceeds a predetermined upper limit value.

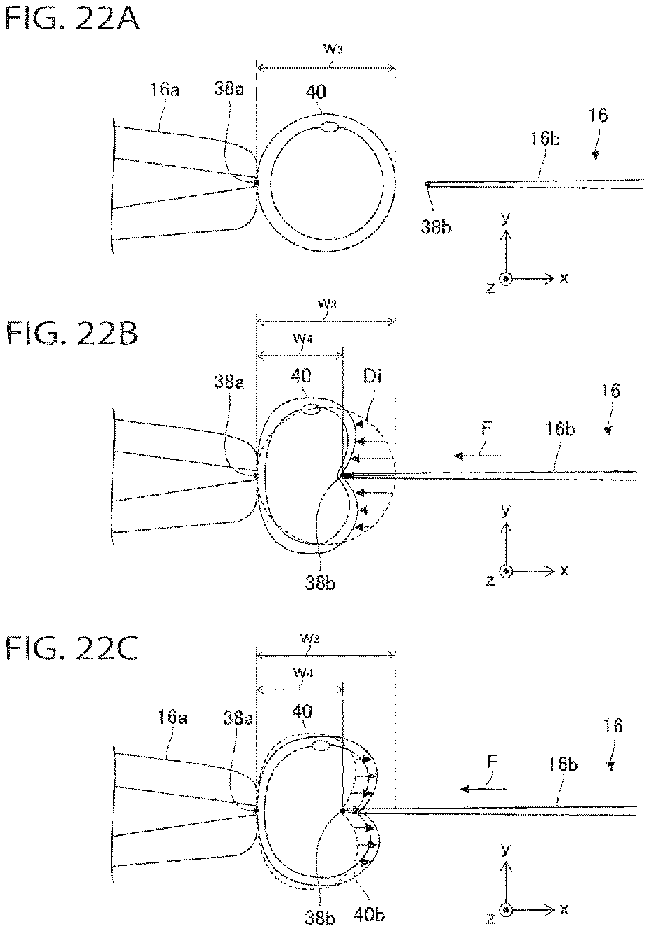

A description will now be given of the flow of piercing operation on the sample 40 by the manipulation system 210. FIGS. 22A-22C are diagrams schematically showing a piercing operation on the sample 40 by the manipulator 16.

FIG. 22A shows a state in which the sample 40 is held by the holding pipette 16a and shows a state before the start of the piercing operation by the injection pipette 16b. FIG. 22A shows that the sample 40 is not deformed by being manipulated by the injection pipette 16b and corresponds to the start point t=0 of the piercing operation on the sample 40. The control apparatus 30 acquires a captured image as shown in FIG. 22A and begins calculating the optical flow of the sample 40.

FIG. 22B shows a state in which the sample 40 is being pierced by the injection pipette 16b and shows a state immediately before the sample 40 is pierced. The sample 40 is deformed as if it is crushed in the x direction in response a force F applied in the x direction by the leading end 38b of the injection pipette 16b. The size of the sample 40 in the x direction is reduced from w3 before the piercing operation to w4 during the piercing operation. The control apparatus 30 calculates the amount of deformation Di at a plurality of positions of the sample 40 from the optical flow of the sample 40 calculated based on the captured image in the process of piercing operation and calculates the air pressure $Pi=\beta Di$ according to the amount of deformation Di. The third force sensation presentation apparatus 100 drives the plurality of actuators 104a-104g based on the air pressure Pi calculated by the control apparatus 30 and presents a planar force sensation to the user.

FIG. 22C shows a state immediately after the piercing of the sample 40 by the injection pipette 16b. The leading end 38b of the injection pipette 16b reaches the interior of the sample 40. When the sample 40 is pierced and the zona pellucida 40b is torn, the zona pellucida 40b of the sample 40 is no longer pressed by the leading end 38b of the injection pipette 16b, and the sample 40 tries to return to its original size. As a result, the amount of deformation Di calculated by the control apparatus 30 becomes smaller than it was immediately before the piercing in FIG. 22B, and the air pressure $Pi=\beta Di$ calculated by the control apparatus 30 also becomes smaller. The third force sensation presentation apparatus 100 presents to the user a planar force sensation in which the magnitude of force is smaller as compared to what it was immediately before the piercing in FIG. 22B. The user can know through a force sensation that the sample 40 has been pierced, by experiencing a decrease in tightening during the piercing operation on the sample 40 through the third force sensation presentation apparatus 100.

The control apparatus 30 may evaluate the extensibility of the sample 40 based on the captured image. The extensibility CE of the sample 40 can be given by $CE=(w3-w4)/w3$ by using the size w3 of the sample 40 in the x direction before the piercing operation and the size w4 of the sample 40 in the x direction immediately before the piercing. w3-w4 represents the amount of deformation D from before the piercing operation on the sample 40 to just before the piercing. The extensibility CE can also be said to be a deformation ratio, which is a proportion of the amount of deformation D with reference to the size of the sample 40.

The control apparatus 30 can identify the sizes w3, w4 of the sample 40 by using the captured images. The size w4 of the sample 40 in the x direction immediately before the piercing may be calculated by referring to the coordinates of the leading end 38a of the holding pipette 16a and the leading end 38b of the injection pipette 16b and finding a distance between the coordinates. The control apparatus 30 can identify the moment of piercing by detecting a reversal of the direction of deformation of the sample 40 in a state where the force F is applied from the injection pipette 16b to the sample 40 in the piercing direction (-x direction). For example, the control apparatus 30 identifies the time when the amount of deformation ΔDi of the sample 40 in the x direction calculated based on the optical flow changes from the -x direction to the +x direction as the moment of piercing.

The control apparatus 30 may determine whether the extensibility is proper depending on whether the calculated extensibility CE exceeds a predetermined threshold value and may output a determination result. The control apparatus 30 may output a determination result indicating that the extensibility is proper when the calculated extensibility CE exceeds a predetermined threshold value (e.g., 0.7). The control apparatus 30 may output a determination result indicating that the extensibility is poor when the calculated extensibility CE is equal to or less than a predetermined threshold value (e.g., 0.7). The control apparatus 30 may output the calculated numerical value of the extensibility CE. In this case, the extensibility CE of the sample 40 is automatically evaluated by the control apparatus 30 so that even a beginner with little experience in cell manipulation can properly evaluate the extensibility CE of the sample 40 during the piercing operation.

Given above is a description of the present disclosure based on the embodiments. The present disclosure is not restricted by the embodiments described above, and it will be understood by those skilled in the art that various design changes are possible and various modifications are possible and that such modifications are also within the scope of the present disclosure.

INDUSTRIAL APPLICABILITY

According to the present disclosure, the operability experienced when a sample is manipulated by using a manipulator is improved.

REFERENCE SIGNS LIST

10, 110, 210 ... manipulation system, 12 ... stage, 16 ... manipulator, 16a ... holding pipette, 16b ... injection pipette, 20 ... objective lens, 22 ... variable focus lens, 24 ... imaging apparatus, 26 ... manipulator drive mechanism, 28 ... lens drive mechanism, 30 ... control apparatus, 32 ... display apparatus, 34 ... input apparatus, 36 ... force sensation presentation apparatus, 38a, 38b ... leading end, 40 ... sample, 78 ... pump, 80 ... second force sensation presentation apparatus, 84 ... second holding member, 86 ... rotating member, 100 ... third force sensation presentation apparatus, 102 ... immobilizing brace, 104a-104g... actuator, 106 ... drive control apparatus

**Claims**

1. A manipulation system comprising:

   a manipulator for manipulating a sample;
   a manipulator drive mechanism for moving the manipulator;
   an imaging apparatus for imaging the sample through an objective lens;
   a control apparatus that generates force information indicating a magnitude of a force sensation presented to a user, based on an image captured by the imaging apparatus; and
   a force sensation presentation apparatus configured to receive an input operation from the user for designating a position of the manipulator and to present a force sensation according to the force information generated by the control apparatus to the user.

2. The manipulation system according to CLAIM 1,
   wherein the control apparatus identifies, based on the image, an amount of change in at least one of a position or a shape of the sample and generates the force information based on the amount of change identified.

3. The manipulation system according to CLAIM 2,
   wherein the control apparatus identifies, based on the image, an amount of deformation of the sample in a direction of protrusion of a leading end of the manipulator and generates the force information based on the amount of deformation.

4. The manipulation system according to CLAIM 3,
   wherein the control apparatus identifies the amount of deformation based on a position of the leading end of the manipulator, a central position of the sample, and a position of an end of the sample in the direction of protrusion.

5. The manipulation system according to CLAIM 2,
   wherein the control apparatus identifies an amount of displacement of the sample based on the image and generates the force information based on the amount of displacement.

6. The manipulation system according to any one of CLAIMS 1 through 5,
   wherein the control apparatus estimates a force applied between the sample and the manipulator based on the image and generates the force information indicating a force derived from amplifying a magnitude of the force thus estimated by 100 times or more.

**7.** The manipulation system according to CLAIM 1, further comprising:

a pump that variably controls a suction force and a discharge force at a leading end of the manipulator,
wherein the force sensation presentation apparatus is configured to further receive an input operation from the user for designating a suction force and a discharge force of the pump, and
wherein the control apparatus acquires manipulation information indicating a magnitude of manipulation designating the suction force and the discharge force of the pump from the force sensation presentation apparatus and generates the force information based on a position of the sample with respect to a position of the leading end of the manipulator identified based on the image and on the manipulation information.

**8.** The manipulation system according to CLAIM 1, further comprising:

a pump that variably controls a suction force and a discharge force at a leading end of the manipulator,
wherein the force sensation presentation apparatus includes a holding member held by the user, a link mechanism that supports the holding member so that a position of the holding member is variable according to a user operation, a position sensor for detecting the position of the holding member, a rotating member that rotates with respect to the holding member according to a gripping action of the user, an angle sensor for detecting a rotation angle of the rotating member, and an actuator that applies a reaction force to the rotating member according to the force information,
wherein the control apparatus controls an operation of the manipulator drive mechanism based on position information indicating the position of the holding member detected by the position sensor and controls an operation of the pump based on angle information indicating the rotation angle of the rotating member detected by the angle sensor, and
wherein the control apparatus generates the force information based on a position of the sample with respect to a position of the leading end of the manipulator identified based on the image and on the angle information.

**9.** The manipulation system according to CLAIM 7 or 8,

wherein the control apparatus determines whether the sample is in contact with the leading end of the manipulator by referring to the position of the sample with respect to the position of the leading end of the manipulator identified based on the image, and
wherein, when the control apparatus determines that the sample is in contact with the leading end of the manipulator, the control apparatus generates the force information indicating a force larger than that of a case where the control apparatus determines that the sample is not in contact with the leading end of the manipulator.

**10.** The manipulation system according to any one of CLAIMS 1 through 9, further comprising:

a further force sensation presentation apparatus configured to present a force sensation in which a magnitude of force differs in each of a plurality of locations on a surface of a user's body,
wherein the control apparatus identifies, based on the image, an amount of deformation of the sample in a direction of protrusion of a leading end of the manipulator at each of a plurality of different positions in a direction perpendicular to the direction of protrusion and generates a plurality of items of force information corresponding to the amount of deformation of the sample at the plurality of positions, and
wherein the further force sensation presentation apparatus presents a force sensation at each of the plurality of locations on the surface of the user's body.

**11.** The manipulation system according to any one of CLAIMS 1 through 10,
wherein the control apparatus detects a reversal of a direction of deformation of the sample in a state where a force is applied from the manipulator to the sample in a direction of protrusion of a leading end of the manipulator and evaluates extensibility of the sample based on the amount of deformation of the sample identified when the reversal is detected.

**12.** A manipulation method comprising:

acquiring position information based on a user's input operation for designating a position of a manipulator from a force sensation presentation apparatus;
controlling, based on the position information acquired, an operation of a manipulator drive mechanism for moving the manipulator;

imagining a sample manipulated by using the manipulator through an objective lens;

generating, based on an image captured, force information indicating a magnitude of a force sensation presented to a user; and

controlling an operation of the force sensation presentation apparatus so that a force sensation according to the force information generated is presented to the user.

**13.** A program comprising computer-implemented modules including:

a module that acquires position information based on a user's input operation for designating a position of the manipulator from a force sensation presentation apparatus;

a module that controls, based on the position information acquired, an operation of a manipulator drive mechanism for moving the manipulator;

a module that acquires an image of a sample manipulated by using the manipulator captured through an objective lens;

a module that generates, based on the image acquired, force information indicating a magnitude of a force sensation presented to a user; and

a module that controls an operation of the force sensation presentation apparatus so that a force sensation according to the force information generated is presented to the user.

**14.** A manipulation system comprising:

a manipulator for manipulating the sample;

a manipulator drive mechanism for moving the manipulator;

a pump that variably controls a suction force and a discharge force at a leading end of the manipulator;

an input operation apparatus including a holding member held by a user, a link mechanism that supports the holding member so that a position of the holding member is variable according to a user operation, a position sensor for detecting the position of the holding member, a rotating member that rotates with respect to the holding member according to a gripping action of the user, and an angle sensor for detecting a rotation angle of the rotating member; and

a control apparatus that controls an operation of the manipulator drive mechanism based on position information indicating the position of the holding member detected by the position sensor and controls an operation of the pump based on angle information indicating the rotation angle of the rotating member detected by the angle sensor.

**15.** A manipulation method comprising:

acquiring position information indicating a position of a holding member held by a user and supported by a link mechanism so that the position is variable according to a user operation;

acquiring angle information indicating a rotation angle of a rotating member that rotates with respect to the holding member according to a gripping action of the user;

controlling, based on the position information acquired, an operation of a manipulator drive mechanism for moving a manipulator for manipulating a sample; and

controlling an operation of a pump that variably controls a suction force and a discharge force at a leading end of the manipulator, based on the angle information acquired.

**16.** A program comprising computer-implemented modules including:

a module that acquires position information indicating a position of a holding member held by a user and supported by a link mechanism so that the position is variable according to a user operation;

a module that acquires angle information indicating a rotation angle of a rotating member that rotates with respect to the holding member according to a gripping action of the user;

a module that controls, based on the position information acquired, an operation of a manipulator drive mechanism for moving a manipulator for manipulating a sample; and

a module that controls an operation of a pump that variably controls a suction force and a discharge force at a leading end of the manipulator, based on the angle information acquired.

# FIG. 1

FIG. 2

FIG. 3

FIG. 4

FIG. 5

FIG. 6A

FIG. 6B

FIG. 6C

FIG. 6D

FIG. 6E

FIG. 6F

FIG. 6G

FIG. 6H

FIG. 6I

FIG. 6J

# FIG. 7

```
                    ┌─────────┐
                    │  START  │
                    └─────────┘
                         │
  S10    ┌───────────────────────────────┐
         │    CALIBRATE ORIGIN OF         │
         │       MANIPULATOR             │
         └───────────────────────────────┘
                         │
                         ▼
  S12    ┌───────────────────────────────┐
         │     ACQUIRE CAPTURED IMAGE     │
         └───────────────────────────────┘
                         │
  S14              ◇───────────────◇         N
         │    IS SAMPLE FOUND?    │────────┐
                   ◇───────────────◇
                         │ Y
  S16    ┌───────────────────────────────┐
         │ ACQUIRE MULTIPLE CAPTURED IMAGES│
         │ BY CHANGING WORKING DISTANCE   │
         └───────────────────────────────┘
                         │
  S18    ┌───────────────────────────────┐
         │   IDENTIFY THREE-DIMENSIONAL   │
         │     POSITION OF SAMPLE        │
         └───────────────────────────────┘
                         │
  S20    ┌───────────────────────────────┐
         │   IDENTIFY THREE-DIMENSIONAL   │
         │   POSITION OF MANIPULATOR     │
         └───────────────────────────────┘
                         │
  S22    ┌───────────────────────────────┐
         │   MAP SAMPLE AND MANIPULATOR   │
         │      TO VIRTUAL SPACE         │
         └───────────────────────────────┘
                         │
  S24    ┌───────────────────────────────┐
         │   DISPLAY THREE-DIMENSIONAL    │
         │   RELATIVE ARRANGEMENT OF      │
         │   SAMPLE AND MANIPULATOR      │
         └───────────────────────────────┘
                         │
                         ▼
  S26              ◇───────────────◇
         │         END?          │
    N    ◇───────────────◇
                         │ Y
                    ┌─────────┐
                    │   END   │
                    └─────────┘
```

FIG. 8A

FIG. 8B

FIG. 8C

# FIG. 9

```
                    ┌─────────────┐
                    │    START    │
                    └─────────────┘
                           │
S30                        │
┌──────────────────────────────────────────┐
│      ACQUIRE POSITION INFORMATION BASED    │
│      ON INPUT OPERATION FOR DESIGNATING     │
│           MANIPULATOR POSITION              │
└──────────────────────────────────────────┘
                           │
S32                        │
┌──────────────────────────────────────────┐
│      CONTROL MANIPULATOR POSITION BASED     │
│       ON ACQUIRED POSITION INFORMATION      │
└──────────────────────────────────────────┘
                           │
S34                        │
┌──────────────────────────────────────────┐
│       ACQUIRE IMAGE CAPTURING SAMPLE        │
│       MANIPULATED BY USING MANIPULATOR      │
└──────────────────────────────────────────┘
                           │
S36                        │
┌──────────────────────────────────────────┐
│          IDENTIFY AMOUNT OF CHANGE          │
│       IN POSITION OR SHAPE OF SAMPLE        │
│           BASED ON ACQUIRED IMAGE           │
└──────────────────────────────────────────┘
                           │
S38                        │
┌──────────────────────────────────────────┐
│       ESTIMATE FORCE APPLIED BETWEEN        │
│      SAMPLE AND MANIPULATOR BASED ON        │
│          IDENTIFIED AMOUNT OF CHANGE        │
└──────────────────────────────────────────┘
                           │
S40                        │
┌──────────────────────────────────────────┐
│  PRESENT FORSE SENSATION CORRESPONDING      │
│     TO ESTIMATED FORCE TO USER THROUGH      │
│ FORCE SENSATION PRESENTATION APPARATUS      │
└──────────────────────────────────────────┘
                           │
                    ┌─────────────┐
                    │   RETURN    │
                    └─────────────┘
```

FIG. 10

FIG. 11A

FIG. 11B

FIG. 12

## FIG. 13

## FIG. 14

## FIG. 15

# FIG. 16

```
                    START

S50
    ACQUIRE POSITION INFORMATION BASED
    ON INPUT OPERATION FOR DESIGNATING
         MANIPULATOR POSITION

S52
         MOVE MANIPULATOR BASED
    ON ACQUIRED POSITION INFORMATION

S54
      ACQUIRE IMAGE CAPTURING SAMPLE
    MANIPULATED BY USING MANIPULATOR

S56
   GENERATE FORCE INFORMATION INDICATING
  MAGNITUDE OF FORCE SENSATION PRESENTED
      TO USER, BASED ON CAPTURED IMAGE

S58
      PRESENT FORSE SENSATION ACCORDING TO
  GENERATED FORCE INFORMATION TO USER THROUGH
     FORCE SENSATION PRESENTATION APPARATUS

                   RETURN
```

# FIG. 17

```
                    ┌──────────┐
                    │  START   │
                    └──────────┘
                         │
S70 ┌────────────────────────────────────────┐
    │ ACQUIRE POSITION INFORMATION INDICATING │
    │      POSITION OF HOLDING MEMBER         │
    └────────────────────────────────────────┘
                         │
S72 ┌────────────────────────────────────────┐
    │   ACQUIRE ANGLE INFORMATION INDICATING  │
    │   ROTATION ANGLE OF ROTATING MEMBER     │
    └────────────────────────────────────────┘
                         │
S74 ┌────────────────────────────────────────┐
    │         MOVE MANIPULATOR BASED          │
    │         ON POSITION INFORMATION         │
    └────────────────────────────────────────┘
                         │
S76 ┌────────────────────────────────────────┐
    │ CONTROL SUCTION FORCE AND DISCHARGE     │
    │ FORCE AT LEADING END OF MANIPULATOR     │
    │      BASED ON ANGLE INFORMATION         │
    └────────────────────────────────────────┘
                         │
S78 ┌────────────────────────────────────────┐
    │  ACQUIRE IMAGE CAPTURING SAMPLE         │
    │  MANIPULATED BY MANIPULATOR AND         │
    │            MANIPULATOR                   │
    └────────────────────────────────────────┘
                         │
S80 ┌────────────────────────────────────────┐
    │   MAKE DETERMINATION ON CONTACT         │
    │ BETWEEN SAMPLE AND MANIPULATOR          │
    │      BASED ON CAPTURED IMAGE            │
    └────────────────────────────────────────┘
                         │
S82                  ◇ CONTACT? ◇─────── N
                         │ Y              │
S84 ┌────────────────────────────────┐  S86 ┌────────────────────────────────┐
    │ GENERATE FORCE INFORMATION     │      │ GENERATE FORCE INFORMATION     │
    │ INDICATING MAGNITUDE OF FORCE  │      │ INDICATING MAGNITUDE OF FORCE  │
    │ OF VALUE OBTAINED BY MULTIPLYING│     │ OF VALUE OBTAINED BY MULTIPLYING│
    │ ROTATION ANGLE BY SECOND       │      │ ROTATION ANGLE BY FIRST        │
    │ COEFFICIENT                    │      │ COEFFICIENT                    │
    └────────────────────────────────┘      └────────────────────────────────┘
                         │◄──────────────────────┘
S88 ┌────────────────────────────────────────┐
    │ PRESENT FORSE SENSATION ACCORDING       │
    │ TO GENERATED FORCE INFORMATION TO       │
    │  USER THROUGH ROTATING MEMBER           │
    └────────────────────────────────────────┘
                         │
                    ┌──────────┐
                    │  RETURN  │
                    └──────────┘
```

## FIG. 18

# FIG. 19

FIG. 20

# FIG. 21

FIG. 22A

FIG. 22B

FIG. 22C

## INTERNATIONAL SEARCH REPORT

| International application No. |
|---|
| **PCT/JP2022/031821** |

| A. | CLASSIFICATION OF SUBJECT MATTER |
|---|---|

*G02B 21/32*(2006.01)i; *B25J 7/00*(2006.01)i; *B25J 13/08*(2006.01)i; *C12M 1/00*(2006.01)i; *C12M 3/00*(2006.01)i
FI: G02B21/32; B25J7/00; B25J13/08 A; C12M1/00 A; C12M3/00 Z

According to International Patent Classification (IPC) or to both national classification and IPC

| B. | FIELDS SEARCHED |
|---|---|

Minimum documentation searched (classification system followed by classification symbols)

G02B21/32; B25J7/00; B25J13/08; C12M1/00; C12M3/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2013-50665 A (NATIONAL UNIV CORP SHIZUOKA UNIV) 14 March 2013 (2013-03-14)<br>paragraphs [0024]-[0057], fig. 1-2 | 1-16 |
| A | JP 2004-255493 A (IBARAKI UNIV) 16 September 2004 (2004-09-16)<br>paragraphs [0017]-[0031], fig. 1 | 1-16 |
| A | JP 2020-122898 A (NSK LTD) 13 August 2020 (2020-08-13)<br>paragraphs [0031]-[0134], fig. 1 | 1-16 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 September 2022** | **27 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2022/031821** |

| Box No. III Observations where unity of invention is lacking (Continuation of item 3 of first sheet) |
| --- |

This International Searching Authority found multiple inventions in this international application, as follows:

Claims are divided into the following two inventions.

(Invention 1) Claims 1-13
Claim 1 has the special technical feature of a "control device for generating force information that represents the magnitude of force to be presented to a user, on the basis of an image captured by the image capturing device," and claims 2-13 have the same special technical feature.
Thus, claims 1-13 are classified as invention 1.

(Invention 2) Claims 14-16
It cannot be said that claims 14-16 have a special technical feature identical or corresponding to that of claim 1 classified as invention 1.
Furthermore, claims 14-16 are not substantially identical to or similarly closely related to any of the claims classified as invention 1.
Thus, claims 14-16 cannot be classified as invention 1.
Also, claim 14 has the special technical feature of an "input operation device comprising: a holding member held by a user; a link mechanism for supporting the holding member such that the position of holding member can be changed depending on the operation of the user; a position sensor for detecting the position of the holding member; a pivoting member that pivots about the holding member by the gripping operation of the user; an angular sensor for detecting a pivot angle of the pivoting member," and claims 15-16 have the same special technical feature. Therefore, claims 14-16 are classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

| Patent document cited in search report | | Publication date (day/month/year) | Patent family member(s) | Publication date (day/month/year) |
|---|---|---|---|---|
| JP | 2013-50665 A | 14 March 2013 | (Family: none) | |
| JP | 2004-255493 A | 16 September 2004 | (Family: none) | |
| JP | 2020-122898 A | 13 August 2020 | (Family: none) | |

**INTERNATIONAL SEARCH REPORT**
Information on patent family members

International application No.

**PCT/JP2022/031821**

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020122898 A

- WO 2008003416 A

**Non-patent literature cited in the description**

- **Y. SUN ; K. T. WAN ; K. P. ROBERTS ; J. C. BISCHOF ; B. J. NELSON.** Mechanical property characterization of mouse zona pellucida. *IEEE Trans Nanobioscience,* 2003, vol. 2 (4), 279-286